# FASCICULE DE BREVET EUROPEEN

(11) **EP 2 542 694 B1**
(45) Date de publication et mention de la délivrance du brevet: **04.11.2015**
(21) Numéro de dépôt: 11714349.5
(22) Date de dépôt: 07.03.2011
(51) Int. Cl.: C12Q 1/68, A61Q 19/00

(54) **MÉTHODE D'ÉVALUATION DU POTENTIEL SENSIBILISANT D'UN COMPOSÉ TEST**
VERFAHREN ZUR SCHÄTZUNG DES SENSIBILISIERUNGSPOTENTIAL EINER TESTVERBINDUNG
METHOD FOR EVALUATING THE SENSIBILISATION POTENTIAL OF A TEST COMPOUND

(30) Priorité: 05.03.2010 FR 1051636
(43) Date de publication de la demande: 09.01.2013
(73) Titulaire: Immunosearch, 06130 Le Plan De Grasse (FR)
(72) Inventeur: GROUX, Hervé, F-06650 Le Rouret (FR); COTTREZ, Françoise, F-06650 Le Rouret (FR)
(74) Mandataire: Regimbeau
(86) Numéro de dépôt international: PCT/FR2011/000122
(87) Numéro de publication internationale: WO 2011/107679

(56) Documents cités:
- WO-A1-2007/090575
- WO-A2-02/070729
- MITSUI GAKU ET AL: "Kinetic profiles of sequential gene expressions for chemokines in mice with contact hypersensitivity.", IMMUNOLOGY LETTERS, vol. 86, no. 2, 3 avril 2003 (2003-04-03), pages 191-197, XP002604478, ISSN: 0165-2478
- BORLON ET AL: "The usefulness of toxicogenomics for predicting acute skin irritation on in vitro reconstructed human epidermis", TOXICOLOGY, vol. 241, no. 3, 23 octobre 2007 (2007-10-23), pages 157-166, XP022310517, LIMERICK, IR ISSN: 0300-483X, DOI: 10.1016/J.TOX.2007.08.096
- NIWA MAKOTO ET AL: "Evaluation of the Skin Irritation Using a DNA Microarray on a Reconstructed Human Epidermal Model", BIOLOGICAL & PHARMACEUTICAL BULLETIN (OF JAPAN), vol. 32, no. 2, 1 février 2009 (2009-02-01), pages 203-208, XP002603918, PHARMACEUTICAL SOCIETY OF JAPAN, TOKYO, JP ISSN: 0918-6158 [extrait le 2008-12-01]
- FLECTCHER S ET AL: "Gene expression analysis of EpiDermTM following exposure to SLS using cDNA microarrays", TOXICOLOGY IN VITRO, vol. 15, no. 4/5, 1 janvier 2001 (2001-01-01), pages 393-398, XP007915270, ELSEVIER SCIENCE, GB ISSN: 0887-2333
- FRIEDMANN P S: "The relationships between exposure dose and response in induction and elicitation of contact hypersensitivity in humans", BRITISH JOURNAL OF DERMATOLOGY, vol. 157, no. 6, décembre 2007 (2007-12), pages 1093-1102, XP002636187, ISSN: 0007-0963
- T. BELLÓN ET AL: "Differential gene expression in drug hypersensitivity reactions: induction of alarmins in severe bullous diseases", BRITISH JOURNAL OF DERMATOLOGY, vol. 162, no. 5, 25 février 2010 (2010-02-25), pages 1014-1022, XP55007187, ISSN: 0007-0963, DOI: 10.1111/j.1365-2133.2009.09627.x
- SEO SEONG JUN ET AL: "Expression of neutrophil gelatinase-associated lipocalin in skin epidermis", JOURNAL OF INVESTIGATIVE DERMATOLOGY, NATURE PUBLISHING GROUP, GB, vol. 126, no. 2, 1 février 2006 (2006-02-01), pages 510-512, XP002559163, ISSN: 0022-202X, DOI: 10.1038/SJ.JID.5700035 [extrait le 2005-12-29]
- PYTHON F ET AL: "Comparative DNA microarray analysis of human monocyte derived dendritic cells and MUTZ-3 cells exposed to the moderate skin sensitizer cinnamaldehyde", TOXICOLOGY AND APPLIED PHARMACOLOGY, ACADEMIC PRESS, AMSTERDAM, NL, vol. 239, no. 3, 15 September 2009 (2009-09-15), pages 273-283, XP026520115, ISSN: 0041-008X, DOI: 10.1016/J.TAAP.2009.06.003 [retrieved on 2009-06-12]

### SEQUENCE LISTING

<110> IMMUNOSEARCH
<120> METHODE D'EVALUATION DU POTENTIEL SENSIBILISANT D'UN COMPOSE TEST
<130> 357910D28420
<150> FR1051636
   <151> 2010-03-05
<160> 82
<170> PatentIn version 3.3
<210> 1
   <211> 24
   <212> DNA
   <213> artificial
<220>
   <223> Amorce sens BRAK
<400> 1
   aagtacccgc actgcgagga gaag 24
<210> 2
   <211> 24
   <212> DNA
   <213> artificial
<220>
   <223> Amorce antisens BRAK
<400> 2
   ttggtgctct gcagcttggg gtgc 24
<210> 3
   <211> 24
   <212> DNA
   <213> artificial
<220>
   <223> Amorce sens CTSS
<400> 3
   tcatacgatc tgggcatgaa ccac 24
<210> 4
   <211> 24
   <212> DNA
   <213> artificial
<220>
   <223> Amorce antisens CTSS
<400> 4
   tgggaactct cagggaactc atca 24
<210> 5
   <211> 24
   <212> DNA
   <213> artificial
<220>
   <223> Amorce sens DAPK2
<400> 5
   tttccttcag catcgtgtcc ctgt 24
<210> 6
   <211> 24
   <212> DNA
   <213> artificial
<220>
   <223> Amorce antisens DAPK2
<400> 6
   tgtcactctc acagttcctc aggt 24
<210> 7
   <211> 24
   <212> DNA
   <213> artificial
<220>
   <223> Amorce sens FABP4
<400> 7
   actgggccag gaatttgacg aagt 24
<210> 8
   <211> 24
   <212> DNA
   <213> artificial
<220>
   <223> Amorce antisens FABP4
<400> 8
   tttctgcaca tgtaccagga cacc 24
<210> 9
   <211> 23
   <212> DNA
   <213> artificial
<220>
   <223> Amorce sens HSP27
<400> 9
   tgcttcacgc ggaaatacac gct 23
<210> 10
   <211> 24
   <212> DNA
   <213> artificial
<220>
   <223> Amorce antisens HSP27
<400> 10
   ctgggatggt gatctcgttg gact 24
<210> 11
   <211> 24
   <212> DNA
   <213> artificial
<220>
   <223> Amorce sens IL18
<400> 11
   tcattgacca aggaaatcgg cctc 24
<210> 12
   <211> 23
   <212> DNA
   <213> artificial
<220>
   <223> Amorce antisens IL18
<400> 12
   agccatacct ctaggctggc tat 23
<210> 13
   <211> 23
   <212> DNA
   <213> artificial
<220>
   <223> Amorce sens IL1R2
<400> 13
   gccagccttg caggaggact ctg 23
<210> 14
   <211> 24
   <212> DNA
   <213> artificial
<220>
   <223> Amorce antisens IL1R2
<400> 14
   ttgcgggtat gagatgaacg gcag 24
<210> 15
   <211> 24
   <212> DNA
   <213> artificial
<220>
   <223> Amorce sens TPSAB1
<400> 15
   acggcccata ctggatgcac ttct 24
<210> 16
   <211> 23
   <212> DNA
   <213> artificial
<220>
   <223> Amorce antisens TPSAB1
<400> 16
   cagcagctgg tcctggtagt aga 23
<210> 17
   <211> 22
   <212> DNA
   <213> artificial
<220>
   <223> Amorce sens CXCR1
<400> 17
   tatgaatctg tccctgccct tc 22
<210> 18
   <211> 24
   <212> DNA
   <213> artificial
<220>
   <223> Amorce antisens CXCR1
<400> 18
   acctcatagc aaactggact ggaa 24
<210> 19
   <211> 24
   <212> DNA
   <213> artificial
<220>
   <223> Amorce sens DEFB1
<400> 19
   ttcctgaaat cctgggtgtt gcct 24
<210> 20
   <211> 24
   <212> DNA
   <213> artificial
<220>
   <223> Amorce antisens DEFB1
<400> 20
   aggcctgtga gaaagttacc acct 24
<210> 21
   <211> 24
   <212> DNA
   <213> artificial
<220>
   <223> Amorce sens DHFR
<400> 21
   ctcattttct ttccagaagt ctag 24
<210> 22
   <211> 24
   <212> DNA
   <213> artificial
<220>
   <223> Amorce antisens DHFR
<400> 22
   tgccaccaac tatccagacc atgt 24
<210> 23
   <211> 24
   <212> DNA
   <213> artificial
<220>
   <223> Amorce sens EHF
<400> 23
   ttggctctct catgtccttg gctt 24
<210> 24
   <211> 24
   <212> DNA
   <213> artificial
<220>
   <223> Amorce antisens EHF
<400> 24
   aggtatgaca ctgtggtagg tgct 24
<210> 25
   <211> 24
   <212> DNA
   <213> artificial
<220>
   <223> Amorce sens IVL
<400> 25
   tgcccacaaa gggagaagta ttgc 24
<210> 26
   <211> 24
   <212> DNA
   <213> artificial
<220>
   <223> Amorce antisens IVL
<400> 26
   tctggacact gcgggtggtt attt 24
<210> 27
   <211> 24
   <212> DNA
   <213> artificial
<220>
   <223> Amorce sens KRT4
<400> 27
   tcacatatgt cccttcccag tcca 24
<210> 28
   <211> 24
   <212> DNA
   <213> artificial
<220>
   <223> Amorce antisens KRT4
<400> 28
   tgccgggtgt tggagaagta gttt 24
<210> 29
   <211> 24
   <212> DNA
   <213> artificial
<220>
   <223> Amorce sens MELANA
<400> 29
   tcttactgct catcggctgt tggt 24
<210> 30
   <211> 24
   <212> DNA
   <213> artificial
<220>
   <223> Amorce antisens MELANA
<400> 30
   tgaagagaca ctttgctgtc ccga 24
<210> 31
   <211> 24
   <212> DNA
   <213> artificial
<220>
   <223> Amorce sens NGAL
<400> 31
   gtgagcacca actacaacca gcat 24
<210> 32
   <211> 24
   <212> DNA
   <213> artificial
<220>
   <223> Amorce antisens NGAL
<400> 32
   agttccgaag tcagctcctt ggtt 24
<210> 33
   <211> 24
   <212> DNA
   <213> artificial
<220>
   <223> Amorce sens PDZK11P1
<400> 33
   caatcgcctt tgcagtcaac cact 24
<210> 34
   <211> 24
   <212> DNA
   <213> artificial
<220>
   <223> Amorce antisens PDZK11P1
<400> 34
   accaggactc catctgcctt gttt 24
<210> 35
   <211> 24
   <212> DNA
   <213> artificial
<220>
   <223> Amorce sens PI3
<400> 35
   tcttgatcgt ggtggtgttc ctca 24
<210> 36
   <211> 24
   <212> DNA
   <213> artificial
<220>
   <223> Amorce antisens PI3
<400> 36
   gactggctct tgcgctttga cttt 24
<210> 37
   <211> 24
   <212> DNA
   <213> artificial
<220>
   <223> Amorce sens PSME2
<400> 37
   tccctcaatg tggctgactt gact 24
<210> 38
   <211> 24
   <212> DNA
   <213> artificial
<220>
   <223> Amorce antisens PSME2
<400> 38
   tctcattccc agggagaaat ccac 24
<210> 39
   <211> 24
   <212> DNA
   <213> artificial
<220>
   <223> Amorce sens SERPINB3
<400> 39
   actcctgggt ggaaagtcaa acga 24
<210> 40
   <211> 24
   <212> DNA
   <213> artificial
<220>
   <223> Amorce antisens SERPINB3
<400> 40
   actcctgggt ggaaagtcaa acga 24
<210> 41
   <211> 24
   <212> DNA
   <213> artificial
<220>
   <223> Amorce sens AKR1B10
<400> 41
   aagatgataa aggtaatgcc atcg 24
<210> 42
   <211> 24
   <212> DNA
   <213> artificial
<220>
   <223> Amorce antisens AKR1B10
<400> 42
   agcttctcga tctggaagtg gctg 24
<210> 43
   <211> 27
   <212> DNA
   <213> artificial
<220>
   <223> Amorce sens AKR1C1
<400> 43
   gcagaggttc ctaaaagtaa agcttta 27
<210> 44
   <211> 27
   <212> DNA
   <213> artificial
<220>
   <223> Amorce antisens AKR1C1
<400> 44
   acctgctcct cattattgta taaatga 27
<210> 45
   <211> 24
   <212> DNA
   <213> artificial
<220>
   <223> Amorce sens AKR1C2
<400> 45
   cattgcatga ggtctgccag aagg 24
<210> 46
   <211> 24
   <212> DNA
   <213> artificial
<220>
   <223> Amorce antisens AKR1C2
<400> 46
   cttagctgta gcttactgaa gtcg 24
<210> 47
   <211> 24
   <212> DNA
   <213> artificial
<220>
   <223> Amorce sens CYP1B1
<400> 47
   atcaacaagg acctgaccag caga 24
<210> 48
   <211> 24
   <212> DNA
   <213> artificial
<220>
   <223> Amorce antisens CYP1B1
<400> 48
   tcatttgggt tggccctgaa atcg 24
<210> 49
   <211> 24
   <212> DNA
   <213> artificial
<220>
   <223> Amorce sens FTH1P
<400> 49
   ctttgaccgc gatgatgtgg cttt 24
<210> 50
   <211> 24
   <212> DNA
   <213> artificial
<220>
   <223> Amorce antisens FTH1P
<400> 50
   tcagtttctc agcatgttcc ctct 24
<210> 51
   <211> 24
   <212> DNA
   <213> artificial
<220>
   <223> Amorce sens FTL
<400> 51
   ttggatcttc atgccctggg ttct 24
<210> 52
   <211> 24
   <212> DNA
   <213> artificial
<220>
   <223> Amorce antisens FTL
<400> 52
   agtcgtgctt gagagtgagc cttt 24
<210> 53
   <211> 24
   <212> DNA
   <213> artificial
<220>
   <223> Amorce sens G6PD
<400> 53
   gaacctcatg gtgctgagat ttgc 24
<210> 54
   <211> 24
   <212> DNA
   <213> artificial
<220>
   <223> Amorce antisens G6PD
<400> 54
   tgaggataac gcaggcgatg ttgt 24
<210> 55
   <211> 24
   <212> DNA
   <213> artificial
<220>
   <223> Amorce sens GCLM
<400> 55
   atggcctgtt cagtccttgg agtt 24
<210> 56
   <211> 24
   <212> DNA
   <213> artificial
<220>
   <223> Amorce antisens GCLM
<400> 56
   tcccagtaag gctgtaaatg ctcc 24
<210> 57
   <211> 24
   <212> DNA
   <213> artificial
<220>
   <223> Amorce sens NQO2
<400> 57
   cacgaagaca ggagtcaatg gaga 24
<210> 58
   <211> 24
   <212> DNA
   <213> artificial
<220>
   <223> Amorce antisens NQ02
<400> 58
   cggatgcaat ttcaggagca aagc 24
<210> 59
   <211> 24
   <212> DNA
   <213> artificial
<220>
   <223> Amorce sens SLC7A11
<400> 59
   tatccctggc atttggacgc taca 24
<210> 60
   <211> 24
   <212> DNA
   <213> artificial
<220>
   <223> Amorce antisens SLC7A11
<400> 60
   tgcccacagc tgtaatgagc ttga 24
<210> 61
   <211> 24
   <212> DNA
   <213> artificial
<220>
   <223> Amorce sens TXNRD1
<400> 61
   ggtcctcaca ggattaaggc aaca 24
<210> 62
   <211> 24
   <212> DNA
   <213> artificial
<220>
   <223> Amorce antisens TXNRD1
<400> 62
   tgcccaagta acgtggtctt tcac 24
<210> 63
   <211> 24
   <212> DNA
   <213> artificial
<220>
   <223> Amorce sens UGT1A1
<400> 63
   acagaacttt ctgtgcgacg tggt 24
<210> 64
   <211> 24
   <212> DNA
   <213> artificial
<220>
   <223> Amorce antisens UGT1A1
<400> 64
   agccagacag atgcagagct caat 24
<210> 65
   <211> 24
   <212> DNA
   <213> artificial
<220>
   <223> Amorce sens UGT1A9
<400> 65
   gctttgccga ggcagggaag ctac 24
<210> 66
   <211> 24
   <212> DNA
   <213> artificial
<220>
   <223> Amorce antisens UGT1A9
<400> 66
   atgagtttct ccaccaccga cctc 24
<210> 67
   <211> 24
   <212> DNA
   <213> artificial
<220>
   <223> Amorce sens YWHAZ
<400> 67
   acagcagatg gctcgagaat acag 24
<210> 68
   <211> 24
   <212> DNA
   <213> artificial
<220>
   <223> Amorce antisens YWHAZ
<400> 68
   tgctctctgc ttgtgaagca ttgg 24
<210> 69
   <211> 24
   <212> DNA
   <213> artificial
<220>
   <223> Amorce sens CD36
<400> 69
   acagatgcag cctcatttcc acct 24
<210> 70
   <211> 25
   <212> DNA
   <213> artificial
<220>
   <223> Amorce antisens CD36
<400> 70
   gggattcctt tcagattaac gtcgg 25
<210> 71
   <211> 24
   <212> DNA
   <213> artificial
<220>
   <223> Amorce sens CYP1A1
<400> 71
   agtggcagat caaccatgac caga 24
<210> 72
   <211> 24
   <212> DNA
   <213> artificial
<220>
   <223> Amorce antisens CYP1A1
<400> 72
   acaccttgtc gatagcacca tcag 24
<210> 73
   <211> 24
   <212> DNA
   <213> artificial
<220>
   <223> Amorce sens GCLC
<400> 73
   aatgggcaat tgctgtctcc aggt 24
<210> 74
   <211> 24
   <212> DNA
   <213> artificial
<220>
   <223> Amorce antisens GCLC
<400> 74
   aaagggagat gcagcactca aagc 24
<210> 75
   <211> 24
   <212> DNA
   <213> artificial
<220>
   <223> Amorce sens HMOX1
<400> 75
   gggccagcaa caaagtgcaa gatt 24
<210> 76
   <211> 24
   <212> DNA
   <213> artificial
<220>
   <223> Amorce antisens HMOX1
<400> 76
   tcgccaccag aaagctgagt gtaa 24
<210> 77
   <211> 24
   <212> DNA
   <213> artificial
<220>
   <223> Amorce sens NQO1
<400> 77
   agtggctcca tgtactctct gcaa 24
<210> 78
   <211> 24
   <212> DNA
   <213> artificial
<220>
   <223> Amorce antisens NQO1
<400> 78
   ttctccaggc gtttcttcca tcct 24
<210> 79
   <211> 23
   <212> DNA
   <213> artificial
<220>
   <223> Amorce sens S100A8
<400> 79
   gggatgacct gaagaaattg cta 23
<210> 80
   <211> 25
   <212> DNA
   <213> artificial
<220>
   <223> Amorce antisens S100A8
<400> 80
   tgttgatatc caactctttg aacca 25
<210> 81
   <211> 24
   <212> DNA
   <213> artificial
<220>
   <223> Amorce sens HSP90
<400> 81
   tctgcctctg gtgatgagat ggtt 24
<210> 82
   <211> 24
   <212> DNA
   <213> artificial
<220>
   <223> Amorce antisens HSP90
<400> 82
   tttccgaaga cgttccacaa aggc 24

## Description

La présente invention se rapporte à une méthode d'évaluation du potentiel sensibilisant d'un composé test et à une trousse pour la mise en oeuvre de ladite méthode.

Les industries de la parfumerie, de la cosmétique et de la pharmacie se doivent de rester compétitives et performantes et continuer à proposer régulièrement des produits nouveaux, avec comme contrainte de répondre aux normes de sécurité pour l'homme et son environnement attachées à leur utilisation. Or l'allergie de contact est l'un des risques majeurs associés à l'utilisation de tels produits.
L'allergie cutanée de contact (ou dermatite atopique) est un problème de santé publique majeur chez l'homme. Elle représente une manifestation immunotoxique environnementale sérieuse, contraignante, dont il est important d'anticiper les effets lorsque l'on met sur le marché des produits susceptibles de la provoquer. La sensibilisation cutanée et, par conséquent la manifestation allergique associée, est le résultat dans un premier temps de l'interaction d'une molécule allergénique avec des cellules spécialisées de l'épiderme, les cellules présentatrices de l'antigène (cellules de Langerhans, cellules dendritiques) puis dans un second temps de leurs présentations par ces cellules à des lymphocytes effecteurs T CD4⁺ et CD8⁺. Ce sont ces derniers qui sont à la base de la réaction allergique et inflammatoire. Néanmoins, les allergènes, en particulier ceux qui peuvent être présents dans un parfum, sont des petites molécules qui ne peuvent pas être reconnues directement. Leur reconnaissance nécessite leur association préalable avec des protéines du soi. Ainsi, c'est le complexe hétérodimérique néoformé dans la peau qui sera ultérieurement présenté aux cellules T dans les ganglions proximaux. Par conséquent, la faculté d'une molécule chimique (composé de parfum ou ingrédient cosmétique) à s'associer à une protéine de l'utilisateur de ce produit est un préalable obligatoire à l'induction de la réaction pathologique cutanée consécutive. Cette réaction pathologique cutanée pourra soit être une irritation, soit une sensibilisation, soit dans la majorité des cas, à la fois une irritation et une sensibilisation.
L'irritation est une inflammation réversible des tissus vivants par action chimique au site de contact. Celle-ci se reconnaît par un oedème consécutif à un afflux de fluides dans les tissus, une rougeur, de la chaleur et/ou de la douleur. En réponse à une agression chimique, les kératinocytes de l'épiderme et les fibroblastes du derme sont stimulés et vont libérer dans la peau des cytokines IL1, TNF alpha, IL6, IL8, ainsi que des médiateurs comme les prostaglandines (PGE2) qui vont initier la réponse inflammatoire.

Les hypersensibilités retardées et immédiates à la base de la sensibilisation impliquent une notion de « mémoire » ce qui souligne leur caractère irréversible contrairement à l'irritation. Dans un cas comme dans l'autre, les mécanismes se déroulent en deux phases :
- la première, dite phase de sensibilisation, pendant laquelle l'antigène/allergène est présenté au système immunitaire et en particulier aux lymphocytes T qui enregistrent le signal moléculaire et régulent, via les cytokines produites, les autres populations cellulaires impliquées (lymphocytes B, T CD8, cellules endothéliales, macrophages, mastocytes, kératinocytes etc...) ;
- la seconde phase, dite la phase effectrice, pendant laquelle différentes populations cellulaires cutanées vont intervenir par l'intermédiaire de médiateurs chimiques responsables des désordres pathologiques. Dans le cas de l'hypersensibilité immédiate, la génération d'anticorps anaphylactiques comme les IgE, en se fixant sur les mastocytes et les basophiles, va conduire à la libération d'histamine, principal vecteur des manifestations allergiques. Dans le cas de l'hypersensibilité retardée, ce sont des cellules T de type cytotoxique (TCD8) qui en détruisant les kératinocytes sont responsables des lésions cutanées.

Ainsi, même si d'un point de vue histologique les dermatites de contact sensibilisantes et irritantes sont très voisines, les conséquences immunologiques analysées au niveau cellulaire ne sont pas pour autant similaires. Il est par conséquent important de posséder des méthodologies fiables permettant de les distinguer. Une approche prédictive originale est d'autant plus nécessaire qu'actuellement aucune corrélation claire n'a été mise en évidence entre une structure moléculaire donnée et l'allergénécité prise au sens large du terme.

Jusqu'à présent, des animaux étaient utilisés pour identifier les molécules sensibilisantes au niveau cutané et le LLNA (local lymph node assay) basé sur la prolifération induite des lymphocytes ganglionnaires après contact avec le sensibilisant a été développé. Ce test a été adopté comme « Testing guideline 429 » par l'Organisation de coopération et de développement économiques (OCDE) et est considéré encore à ce jour comme le test de référence pour la détermination d'un agent chimique sensibilisant.
Les nouvelles contraintes européennes imposent désormais l'utilisation de méthodes n'utilisant pas d'animaux et il est donc indispensable de mettre au point des méthodes alternatives permettant de déterminer si une composition nouvelle ou un produit nouveau est susceptible de représenter un risque pour l'homme par ses propriétés sensibilisantes.
Les Inventeurs ont montré que la reconnaissance *in vivo* d'une substance ne se fait pas au niveau du ganglion drainant, comme cela est généralement admis, mais bien au niveau du tissu où la substance entre en contact avec l'organisme dans le cas présent : la peau. Cela expliquerait pourquoi certaines substances sont sensibilisantes dans un tissu et pas dans un autre comme cela est souvent observé. Il apparait donc que c'est la réaction du tissu cutané qui instruit le message aux cellules présentatrices de l'allergène : les cellules dendritiques vont alors le transmettre aux lymphocytes T dans le ganglion drainant.

Or, il est généralement admis que les modèles de peau ne sont pas suffisants pour analyser les réponses des sensibilisants et qu'il faut y ajouter des cellules dendritiques (EP 0 857 971).

Les Inventeurs ont maintenant mis en évidence que la peau constitue un modèle suffisant pour mettre en évidence des biomarqueurs spécifiques de la sensibilisation et/ou de l'irritation chez l'homme et chez la souris, et qu'il n'est pas nécessaire d'ajouter d'autres types cellulaires si l'analyse des gènes identifiés est réalisée à un temps donné. Le modèle EPISKIN peut ainsi être le tissu de référence pour évaluer le caractère sensibilisant d'un composant test.

Par ailleurs, les Inventeurs ont montré qu'il n'était pas suffisant de mettre en évidence une surexpression d'un seul desdits biomarqueurs pour conclure au potentiel sensibilisant d'un composé test, et que seule l'étude d'au moins six marqueurs spécifiques de la sensibilisation permettaient de conclure au potentiel sensibilisant du composé test.

Ainsi, la présente invention se rapporte à une méthode d'évaluation du potentiel sensibilisant d'un composé test, comprenant les étapes de:
a) mise en contact d'un composé test avec un échantillon biologique;
b) détermination du niveau d'expression d'au moins six gènes choisis dans le groupe constitué par : BRAK(CXC chimiokine ligand 14), CTSS (cathepsine S), DAPK2 (protéine kinase associée à la mort 2), FABP4 (Proteine de fixation des acides gras 4), HSP27 (Protéine de choc thermique de27kDa), IL18 (Interleukine 18), HSP90 (Protéine de choc thermique de 90kDa), IL1R2 (Recepteur type II de l'interleukine 1), TPSAB1 (tryptase alpha/beta 1), CXCR1 (Recepteur alpha de l'interleukin 8), DEFB1 (defensine, beta 1), DHFR (dihydrofolate reductase), EHF (Facteur homologue ets), IVL (involucrine), KRT4 (keratine 4), MELANA (melane-A), NGAL (Gelatinase des neutrophiles), PDZK11P1 (Protéine d'interaction PDZK1), PI3 (Inhibiteur de peptidase), PSME2 (Sous unité activatrice du proteasome 2), SERPINB3 (Membre 3 des Inhibiteurs de la serpine peptidase), AKR1B10 (Membre B10 de la famille des aldo-keto reductases), AKR1C1 (Membre C1 de la famille des aldo-keto reductases), AKR1C2 (Membre C2 de la famille des aldo-keto reductases), CYP1B1 (cytochrome P450, famille 1, sous famille B, polypeptide 1), FTH1P (Polypeptide lourd de la ferritine, 1), FTL (Polypeptide léger de la ferritine, 1), G6PD (glucose-6-phosphate dehydrogenase), GCLM (Sous unité modificatrice de la glutamate-cysteine ligase), NQO2 (NAD(P)H dehydrogenase des quinone s2), SLC7A11 (Membre 11 de la famille 7 des porteurs de molécules solubles), TXNRD1 (thioredoxine reductase 1), UGT1A1 (Polypeptide A1 de la famille des UDP glucuronosyltransferase 1), UGT1A9 (Polypeptide A9 de la famille des UDP glucuronosyltransferase 1, YWHAZ (Polypeptide zeta de la protéine d'activation de tyrosine 3-monooxygenase/tryptophan 5-monooxygenase polypeptide), CD36 (CD36 molecule), CYP1A1 (cytochrome P450, famille 1, sous famille A, polypeptide 1), GCLC (Sous unité catalitique de la glutamate-cysteine ligase), HMOX1 (heme oxygenase 1), NQO1 (NAD(P)H dehydrogenase,des quinones 1) et S100A8 (Protéine de fixation du calcium S100 A8), caractérisée en ce que l'échantillon de peau est un échantillon de peau reconstruite *in vitro*.

De préférence, lesdits au moins six gènes sont choisis dans le groupe constitué par : BRAK (CXC chimiokine ligand 14), CTSS (cathepsine S), DAPK2 (protéine kinase associée à la mort 2), FABP4 (Proteine de fixation des acides gras 4), HSP27 (Protéine de choc thermique de27kDa), IL18 (Interleukine 18), HSP90 (Protéine de choc thermique de 90kDa), IL1R2 (Recepteur type II de l'interleukine 1), TPSAB1 (tryptase alpha/beta 1), CXCR1 (Recepteur alpha de l'interleukin 8), DEFB1 (defensine, beta 1), DHFR (dihydrofolate reductase), EHF (Facteur homologue ets), IVL (involucrine), KRT4 (keratine 4), MELANA (melane-A), NGAL (Gelatinase des neutrophiles), PDZK11P1 (Protéine d'interaction PDZK1), PI3 (Inhibiteur de peptidase), PSME2 (Sous unité activatrice du proteasome 2), SERPINB3 (Membre 3 des Inhibiteurs de la serpine peptidase), AKR1B10 (Membre B10 de la famille des aldo-keto reductases), AKR1C1 (Membre C1 de la famille des aldo-keto reductases), AKR1C2 (Membre C2 de la famille des aldo-keto reductases), CYP1B1 (cytochrome P450, famille 1, sous famille B, polypeptide 1), FTH1P (Polypeptide lourd de la ferritine, 1), FTL (Polypeptide léger de la ferritine, 1), G6PD (glucose-6-phosphate dehydrogenase), GCLM (Sous unité modificatrice de la glutamate-cysteine ligase), NQO2 (NAD(P)H dehydrogenase des quinone s2), SLC7A11 (Membre 11 de la famille 7 des porteurs de molécules solubles), TXNRD1 (thioredoxine reductase 1), UGT1A1 (Polypeptide A1 de la famille des UDP glucuronosyltransferase 1), UGT1A9 (Polypeptide A9 de la famille des UDP glucuronosyltransferase 1, et YWHAZ (Polypeptide zeta de la protéine d'activation de tyrosine 3-monooxygenase/tryptophan 5-monooxygenase polypeptide),
encore préférentiellement choisis dans le groupe constitué par : BRAK (CXC chimiokine ligand 14), CTSS(cathepsine S), DAPK2 (protéine kinase associée à la mort 2), FABP4 (Proteine de fixation des acides gras 4), HSP27
(Protéine de choc thermique de27kDa), IL18 (Interleukine 18), HSP90 (Protéine de choc thermique de 90kDa), IL1R2 (Recepteur type II de l'interleukine 1), TPSAB1 (tryptase alpha/beta 1), CXCR1 (Recepteur alpha de l'interleukin 8), DEFB1 (defensine, beta 1), DHFR (dihydrofolate reductase), EHF (Facteur homologue ets), IVL (involucrine), KRT4 (keratine 4), MELANA (melane-A), NGAL (Gelatinase des neutrophiles), PDZK11P1 (Protéine d'interaction PDZK1), PI3 (Inhibiteur de peptidase) et SERPINB3 (Membre 3 des Inhibiteurs de la serpine peptidase),
et de manière encore préférée choisis dans le groupe constitué par : BRAK (CXC chimiokine ligand 14), CTSS(cathepsine S), DAPK2 (protéine kinase associée à la mort 2), FABP4 (Proteine de fixation des acides gras 4), HSP27
(Protéine de choc thermique de27kDa), IL18 (Interleukine 18), HSP90 (Protéine de choc thermique de 90kDa), IL1R2 (Recepteur type II de l'interleukine 1), TPSAB1 (tryptase alpha/beta 1) ou dans le groupe constitué par : CXCR1 (Recepteur alpha de l'interleukin 8), DEFB1 (defensine, beta 1), DHFR (dihydrofolate reductase), EHF (Facteur homologue ets), IVL (involucrine), KRT4 (keratine 4), MELANA (melane-A), NGAL (Gelatinase des neutrophiles), PDZK11P1 (Protéine d'interaction PDZK1), PI3 (Inhibiteur de peptidase), PSME2 (Sous unité activatrice du proteasome 2) et SERPINB3 (Membre 3 des Inhibiteurs de la serpine peptidase).

La méthode selon la présente invention comprend en outre une étape c) de détermination du potentiel sensibilisant d'un composé test.

Ladite étape c) consiste en une étape de sélection dudit composé comme présentant un potentiel sensibilisant si le niveau d'expression d'au moins 6 desdits gènes est supérieur à une valeur seuil.

De préférence, la méthode selon la présente invention est une méthode *in vitro*. Tel qu'utilisé ici, le terme « échantillon biologique » se réfère à tout échantillon solide ou liquide issu d'un sujet.

Ledit échantillon biologique est un échantillon de peau.
Selon la présente invention l'échantillon de peau est un échantillon de peau reconstruite *in vitro*, comme par exemple le modèle EpiSkin (EPISKIN, Lyon France), EpiDerm™ (MATEK Corporation, Ashland, MA) ou SkinEthic™ RHE (SKINETHIC, Nice, France). De préférence, ledit échantillon de peau reconstruite *in vitro* comprend en outre une couche de kératine.

De manière encore préférée, l'échantillon de peau ne comprend pas l'addition d'autres types cellulaires supplémentaires, et encore préférentiellement pas de cellules de Langerhans supplémentaires.

Le composé test peut être un composé de nature, structure et origine variées, notamment un composé biologique, un composé chimique, synthétique, etc.

Le composé test peut être tout produit qui se présente sous une forme isolée ou en mélange avec d'autres produits. Le composé test peut être défini en termes de structure et/ou de composition ou être défini sur le plan fonctionnel. Le composé test peut, par exemple, être un produit isolé et structurellement défini, un produit isolé de structure indéfinie, un mélange de produits connus et caractérisés ou une composition comprenant un ou plusieurs produits. Un ou plusieurs composés peuvent ainsi être testés, en mélange ou de manière séparée.

De telles compositions peuvent être, par exemple, des échantillons d'un produit cosmétique ou dermatologique.
De préférence, ledit composé test est apte à être utilisé sur la peau et peut être utilisé dans une composition cosmétique ou dermatologique.

Préférentiellement, ladite méthode permet d'évaluer le potentiel sensibilisant d'un composé test chez l'homme, comprenant une étape b) de détermination du niveau d'expression d'au moins six gènes tels que définis dans le tableau 1, et de préférence six gènes choisis dans le groupe constitué par : CXCR1 (Recepteur alpha de l'interleukin 8), DEFB1 (defensine, beta 1), DHFR (dihydrofolate reductase), EHF (Facteur homologue ets), IVL (involucrine), KRT4 (keratine 4), MELANA (melane-A), NGAL (Gelatinase des neutrophiles), PDZK11P1 (Protéine d'interaction PDZK1), PI3 (Inhibiteur de peptidase), PSME2 (Sous unité activatrice du proteasome 2), SERPINB3 (Membre 3 des Inhibiteurs de la serpine peptidase).

On entend par « potentiel sensibilisant », le risque pour le composé test de provoquer une réaction immunologique lors de sa mise en contact avec un mammifère, de préférence un humain. Ainsi, le potentiel sensibilisant peut être considéré comme le risque de développer une allergie au contact du composé test.

On entend par « potentiel irritant », le risque pour le composé test de provoquer une inflammation réversible des tissus vivants par action chimique au site de contact.

De préférence, la méthode selon la présente invention permet d'évaluer si le composé test est susceptible de provoquer une allergie de contact ou dermatite atopique.

La présente invention est particulièrement adaptée à l'identification d'un nombre important de composés. Ce criblage simple et efficace peut être accompli en un laps de temps très court. Les méthodes décrites peuvent en particulier être partiellement automatisées, autorisant ainsi le criblage efficace et simultané de composés divers et nombreux, soit sous forme de mélange soit sous forme séparée.

De préférence, dans la méthode selon la présente invention, le niveau d'expression de chacun desdits gènes est déterminé par la mesure du niveau d'expression des polypeptides codés par ledit gène ou un fragment de celui-ci, ou par la détermination du niveau d'expression de l'ARNm dudit gène ou d'un fragment de celui-ci.

Dans un mode de réalisation préféré, la détermination du niveau d'expression de chacun desdits au moins six gènes est effectuée par analyse de l'expression de transcrits d'ARNm ou de précurseurs d'ARNm, tel qu'un ARN natif, dudit gène. Ladite analyse peut être réalisée en préparant l'ARNm/ADNc de cellules d'un échantillon biologique d'un patient, et hybridation de l'ARNm /ADNc avec un polynucléotide de référence. L'ARNm/ADNc préparé peut être utilisé dans une analyse par hybridation ou amplification qui inclut, sans s'y limiter, les analyses Southern et Northen, les analyses par PCR (« polymerase chain reaction »), telle que la PCR quantitative (Taqman) et l'utilisation de sondes (« probes arrays ») telles que les matrices ADN GeneChip®(AFFYMETRIX).

Avantageusement, l'analyse de l'expression d'ARNm transcrit de chacun desdits au moins au moins six gènes implique un procédé d'amplification des acides nucléiques, comme par exemple la RT-PCR (mode de réalisation expérimental décrit dans le Brevet US 4,683,202), la réaction en chaîne par la ligase (BARANY, Proc. Natl. Acad. Sci. USA, vol.88, p: 189-193, 1991), la réplication de séquences auto-entretenue (« self sustained séquence réplication ») (GUATELLI et al., Proc. Natl. Acad. Sci. USA, vol.87, p: 1874-1878, 1990), le système d'amplification transcriptionnelle. (KWOH et al., Proc. Natl. Acad. Sci. USA, vol.86, p: 1173-1177, 1989), la « Q-Beta Replicase » (LIZARDI et al., Biol. Technologie, vol.6, p: 1197, 1988), la « rolling circle replication » (U. S. Patent No. 5,854,033) ou toute autre méthode d'amplification d'acides nucléiques, suivie d'une étape de détection des molécules amplifiées par des techniques bien connues de l'homme du métier. Ces modes de détection sont particulièrement utiles pour la détection de molécules d'acides nucléiques en très faibles quantités. Ainsi, selon un mode de réalisation préféré, la méthode selon la présente invention comprend une étape supplémentaire d'amplification de l'ARNm ou de l'ADNc de chacun desdits au moins six gènes, de la séquence complémentaire de celle-ci ou d'un fragment de celle-ci.

Telles qu'utilisées ici, les amorces d'amplifications sont définies comme étant une paire de molécules d'acides nucléiques qui peuvent s'apparier respectivement aux régions 3' et 5' d'un gène de façon spécifique (brins positif et négatif, ou inversement) et encadrent une courte région dudit gène. De manière générale, les amorces d'amplification ont une longueur de 10 à 30 nucléotides et permettent l'amplification d'une région d'une longueur comprise entre 50 et 200 nucléotides. Avantageusement, les amorces utilisées dans le cadre de la présente invention sont celles listées dans le tableau 1.

Dans un autre mode de réalisation préféré, la détermination du niveau d'expression de chacun desdits au moins six gènes est réalisée par détermination du niveau d'expression du polypeptide codé par ledit gène, ou un fragment de celui-ci. Ladite analyse peut être réalisée en utilisant un anticorps (par exemple, un anticorps radiomarqué, marqué avec un chromophore, un fluorophore, ou une enzyme), un dérivé d'anticorps (par exemple un anticorps conjugué à un substrat ou à une protéine ou un ligand d'une protéine d'un couple ligand/protéine (par exemple biotine-streptavidine)) ou un fragment d'anticorps (par exemple un anticorps à une seule chaîne, un domaine hypervariable d'un anticorps isolé, etc.) qui se lie spécifiquement au polypeptide codé par ledit gène. Lesdites analyses peuvent être réalisées par de nombreuses techniques à la portée de l'homme du métier incluant, sans s'y limiter, les tests immunologiques basés sur l'utilisation d'activité enzymatique (« enzyme immunoassay » EIA), les tests immunologiques basés sur l'utilisation d'isotopes radioactifs (RIA), l'analyse par Western blot et les tests ELISA (« enzyme linked immunoabsorbant assay »).

Au sens de la présente invention, on entend par « polypeptide » une séquence comprenant au moins deux acides aminés, et les termes « polypeptide », « peptide » et « protéine » peuvent être indifféremment utilisés.

Au sens de la présente invention, on entend par « fragment de l'ARNm ou de l'ADNc », une séquence d'au moins 50 acides nucléiques, à titre d'exemple d'au moins 100 ou 150 acides nucléiques, de préférence d'au moins 200 acides nucléiques, à titre d'exemple d'au moins 250 ou 350 acides nucléiques, et de manière particulièrement préférée un polypeptide d'au moins 400 acides nucléiques.

Au sens de la présente invention, on entend par « fragment du polypeptide », une séquence d'au moins 50 acides aminés, à titre d'exemple d'au moins 100 ou 150 acides aminés, de préférence d'au moins 200 acides aminés, à titre d'exemple d'au moins 250 ou 350 acides aminés, et de manière particulièrement préférée un polypeptide d'au moins 400 acides aminés.

De préférence, la méthode selon la présente invention comprend en outre une étape de comparaison du niveau d'expression de chacun desdits au moins six gènes avec une valeur de référence. Cette valeur de référence peut servir de contrôle positif et/ou négatif.

Un contrôle positif peut par exemple être effectué en comparant le niveau d'expression dudit au moins un gène en présence du composé test avec le niveau d'expression dudit au moins un gène en présence d'un composé connu comme sensibilisant.

A titre d'exemple de composé connu comme sensibilisant, on peut citer l'acide 2,4,6-trinitrobenzene sulfonique, la p-phénylenediamine, le dinitrochlorobenzène, le benzaldehyde, le résorcinol, le disulfure de tétraméthyl thiurame, l'oxazolone, le chloroAtranol, le diphenylcyclopropénone le potassium dichromate, l'aldéhyde cinnamique, le 2-Bromo-2-(bromomethyl)glutaronitrile, le glyoxal, la saccharine, le formaldehyde, l'anhydride trimellitique, le méthylchloroisothiazolinone, le benzoate de benzyl, l'alpha-hexyl cinnamaldehyde, l'eugenol, le 2-mercaptobenzothiazole, l'soeugenol, la diphénylcyclopropénone (DPCP), le lauryl gallate (LG), la 3-3-dimethylaminopropylamine (3-DMAPA), l'aldéhyde cinnamique (CA), le citral (Cal), la 1,4-hydroquinone (HQ), le glutaraldéhyde (GA), la 1,2-benzisothiazolin-3-one (Ben), la phénylacetaldéhyde (PA) et le lilial (Li), préferentiellement parmi la diphénylcyclopropénone, le lauryl gallate, la 1,4-hydroquinone et le glutaraldéhyde, et de manière particulièrement préférée la 1,4-hydroquinone.

Alternativement, dans le cadre de la présente invention, on peut utiliser une composition sensibilisante comme contrôle positif, comme le « fragrance mix ».

Un contrôle négatif peut être réalisé en l'absence du composé test ou en présence d'un composé connu comme non sensibilisant, comme par exemple l'huile d'olive, le glycérol, le cétyl trimethylammonium bromide (CTAB) et le dipropylène glycol. 1.

Dans le cadre de la présente invention, on conclura qu'un composé test présente un potentiel sensibilisant si une surexpression dudit gène est observée par rapport à son niveau d'expression en l'absence dudit composé test.

On entend par « surexpression », un niveau d'expression significativement plus élevé dudit gène par rapport à son niveau d'expression normal. De préférence, on entend par surexpression un niveau d'expression dans un échantillon biologique qui est supérieur à au moins 20% du niveau normal d'expression dudit gène, de préférence supérieur à au moins 50% du niveau normal d'expression dudit gène, et de manière particulièrement préférée supérieur d'au moins 90% au niveau normal d'expression dudit gène.
Le « niveau d'expression en l'absence dudit composé test » ou « niveau normal » est le niveau d'expression dudit gène dans un échantillon contrôle correspondant potentiellement à l'échantillon biologique d'un patient ne présentant pas de sensibilisation ou, de préférence, à la moyenne du niveau d'expression dudit gène dans différents échantillons contrôle.

De préférence, l'étape b) de ladite méthode d'évaluation du potentiel sensibilisant comprend la mesure de l'expression d'au moins 7, d'au moins 8, d'au moins 9, d'au moins 10, d'au moins 11, d'au moins 12, d'au moins 13, d'au moins 14, d'au moins 15, d'au moins 16, d'au moins 17, d'au moins 18, d'au moins 19, d'au moins 20, d'au moins 21, d'au moins 22, d'au moins 23, d'au moins 24 et encore préférentiellement d'au moins 25 gènes choisis dans le groupe constitué par : les gènes tels que définis dans le tableau 1.
Ainsi, on pourra conclure qu'un composé test présente un potentiel sensibilisant si une surexpression d'au moins 6, d'au moins 7, d'au moins 8, d'au moins 9, d'au moins 10, d'au moins 11, d'au moins 12, d'au moins 13, d'au moins 14, d'au moins 15, d'au moins 16, d'au moins 17, d'au moins 18, d'au moins 19, d'au moins 20, d'au moins 21, d'au moins 22, d'au moins 23, d'au moins 24 et encore préférentiellement d'au moins 25 gènes choisis dans le groupe constitué par les gènes tels que définis dans le tableau 1.

De manière particulièrement préférée, l'étape b) de ladite méthode d'évaluation du potentiel sensibilisant comprend la détermination du niveau d'expression de l'ensemble des gènes : AKR1B10 (Membre B10 de la famille des aldo-keto reductases), AKR1C1 (Membre C1 de la famille des aldo-keto reductases), AKR1C2 (Membre C2 de la famille des aldo-keto reductases), CYP1B1 (cytochrome P450, famille 1, sous famille B, polypeptide 1), FTH1P (Polypeptide lourd de la ferritine, 1), FTL (Polypeptide léger de la ferritine, 1), G6PD (glucose-6-phosphate dehydrogenase), GCLM (Sous unité modificatrice de la glutamate-cysteine ligase), NQO2 (NAD(P)H dehydrogenase des quinone s2), SLC7A11 (Membre 11 de la famille 7 des porteurs de molécules solubles), TXNRD1 (thioredoxine reductase 1), UGT1A1 (Polypeptide A1 de la famille des UDP glucuronosyltransferase 1), UGT1A9 (Polypeptide A9 de la famille des UDP glucuronosyltransferase 1, YWHAZ (Polypeptide zeta de la protéine d'activation de tyrosine 3-monooxygenase/tryptophan 5-monooxygenase polypeptide), CD36 (CD36 molecule), CYP1A1 (cytochrome P450, famille 1, sous famille A, polypeptide 1), GCLC (Sous unité catalitique de la glutamate-cysteine ligase), HMOX1 (heme oxygenase 1), NQO1 (NAD(P)H dehydrogenase,des quinones 1), PSME2 (Sous unité activatrice du proteasome 2) et S100A8 (Protéine de fixation du calcium S100 A8), de préférence choisis dans le groupe constitué par : AKR1B10 (Membre B10 de la famille des aldo-keto reductases), AKR1C1 (Membre C1 de la famille des aldo-keto reductases), AKR1C2 (Membre C2 de la famille des aldo-keto reductases), CYP1B1 (cytochrome P450, famille 1, sous famille B, polypeptide 1), FTH1P (Polypeptide lourd de la ferritine, 1), FTL (Polypeptide léger de la ferritine, 1), G6PD (glucose-6-phosphate dehydrogenase), GCLM (Sous unité modificatrice de la glutamate-cysteine ligase), NQO2 (NAD(P)H dehydrogenase des quinone s2), SLC7A11 (Membre 11 de la famille 7 des porteurs de molécules solubles), TXNRD1 (thioredoxine reductase 1), UGT1A1 (Polypeptide A1 de la famille des UDP glucuronosyltransferase 1), UGT1A9 (Polypeptide A9 de la famille des UDP glucuronosyltransferase 1, PSME2 (Sous unité activatrice du proteasome 2) et YWHAZ (Polypeptide zeta de la protéine d'activation de tyrosine 3-monooxygenase/tryptophan 5-monooxygenase polypeptide).
Dans ce mode de réalisation particulièrement préféré, on conclura au potentiel sensibilisant dudit composé test si au moins 11 desdits gènes sont surexprimés par rapport à une valeur de référence.

De manière particulièrement préférée, l'étape b) de ladite méthode d'évaluation du potentiel sensibilisant comprend la détermination du niveau d'expression d'au moins l'ensemble des gènes : BRAK (CXC chimiokine ligand 14), CTSS (cathepsine S), DAPK2 (protéine kinase associée à la mort 2), FABP4 (Proteine de fixation des acides gras 4), HSP27 (Protéine de choc thermique de27kDa), IL18 (Interleukine 18), HSP90 (Protéine de choc thermique de 90kDa), IL1R2 (Recepteur type II de l'interleukine 1), TPSAB1 (tryptase alpha/beta 1), CXCR1 (Recepteur alpha de l'interleukin 8), DEFB1 (defensine, beta 1), DHFR (dihydrofolate reductase), EHF (Facteur homologue ets), IVL (involucrine), KRT4 (keratine 4), MELANA (melane-A), NGAL (Gelatinase des neutrophiles), PDZK11P1 (Protéine d'interaction PDZK1), PI3 (Inhibiteur de peptidase) et SERPINB3 (Membre 3 des Inhibiteurs de la serpine peptidase),
et encore préférentiellement l'ensemble des gènes BRAK (CXC chimiokine ligand 14), CTSS (cathepsine S), DAPK2 (protéine kinase associée à la mort 2), FABP4 (Proteine de fixation des acides gras 4), HSP27 (Protéine de choc thermique de27kDa), IL18 (Interleukine 18), IL1R2 (Recepteur type II de l'interleukine 1) HSP90 (Protéine de choc thermique de 90kDa), et TPSAB1 (tryptase alpha/beta 1) ou l'ensemble des gènes CXCR1 (Recepteur alpha de l'interleukin 8), DEFB1 (defensine, beta 1), DHFR (dihydrofolate reductase), EHF (Facteur homologue ets), IVL (involucrine), KRT4 (keratine 4), MELANA (melane-A), NGAL (Gelatinase des neutrophiles), PDZK11P1 (Protéine d'interaction PDZK1), PI3 (Inhibiteur de peptidase) et SERPINB3 (Membre 3 des Inhibiteurs de la serpine peptidase).

Dans ce mode de réalisation particulièrement préféré, on conclura au potentiel sensibilisant dudit composé test si au moins 7, de préférence au moins 8 desdits gènes sont surexprimés par rapport à une valeur de référence.

Dans un mode de réalisation particulier, l'étape b) de ladite méthode d'évaluation du potentiel sensibilisant d'un composé test comprend une étape α) de détermination du niveau d'expression d'au moins 10, de préférence 11, 12, 13, 14, 15, 16 et encore préférentiellement de l'ensemble des gènes : AKR1B10 (Membre B10 de la famille des aldo-keto reductases), AKR1C1 (Membre C1 de la famille des aldo-keto reductases), AKR1C2 (Membre C2 de la famille des aldo-keto reductases), CTGF (Facteur de croissance du tissu conjonctif), CYP1B1 (cytochrome P450, famille 1, sous famille B, polypeptide 1), FTH1P (Polypeptide lourd de la ferritine, 1), FTL (Polypeptide léger de la ferritine, 1), G6PD (glucose-6-phosphate dehydrogenase), GCLM (Sous unité modificatrice de la glutamate-cysteine ligase), IER3 (Gène de la réponse précoce immédiate 3), NQO2 (NAD(P)H dehydrogenase des quinone s2), SLC7A11 (Membre 11 de la famille 7 des porteurs de molécules solubles), TXNRD1 (thioredoxine reductase 1), UGT1A1 (Polypeptide A1 de la famille des UDP glucuronosyltransferase 1), UGT1A9 (Polypeptide A9 de la famille des UDP glucuronosyltransferase 1, YWHAZ (Polypeptide zeta de la protéine d'activation de tyrosine 3-monooxygenase/tryptophan 5-monooxygenase polypeptide) et CD36 (CD36 molecule) CYP1A1 (cytochrome P450, famille 1, sous famille A, polypeptide 1), GCLC (Sous unité catalitique de la glutamate-cysteine ligase), HMOX1 (heme oxygenase 1), NQO1 (NAD(P)H dehydrogenase,des quinones 1), S100A8 (Protéine de fixation du calcium S100 A8)
et éventuellement une étape β) de mesure du niveau d'expression d'au moins 10, de préférence 11, 12, 13, 14, 15, 16, 17, 18, 19 et encore préférentiellement de l'ensemble des gènes : BRAK (CXC chimiokine ligand 14), CTSS (cathepsine S), DAPK2 (protéine kinase associée à la mort 2), FABP4 (Protéine de fixation des acides gras 4), HSP27 (Protéine de choc thermique de27kDa), IL18 (Interleukine 18), IL1R2 (Récepteur type II de l'interleukine 1), TPSAB1(tryptase alpha/beta 1), HSP90 (Protéine de choc thermique de 90kDa), CXCR1 (Récepteur alpha de l'interleukin 8), DEFB1 (defensine, beta 1), DHFR (dihydrofolate reductase), EHF (Facteur homologue ets), IVL (involucrine), KRT4 (keratine 4), MELANA (melane-A), NGAL (Gelatinase des neutrophiles), PDZK11P1 (Protéine d'interaction PDZK1), PI3 (Inhibiteur de peptidase), PSME2 (Sous unité activatrice du proteasome 2), SERPINB3 (Membre 3 des Inhibiteurs de la serpine peptidase), et
une étape c) de détermination du potentiel sensibilisant d'un composé test, dans laquelle on conclura au potentiel sensibilisant si :
- le niveau d'expression d'au moins 7 des gènes mesuré à l'étape α) est supérieur à une valeur seuil ; et/ou
- le niveau d'expression d'au moins 7 des gènes mesuré à l'étape β) est supérieur à une valeur seuil.

De préférence, l'étape b) est réalisée entre 2 et 24 heures après l'étape a), de manière encore préférée entre 4 et 18 heures après l'étape a), de manière particulièrement préférée entre 5 et 7 heures après l'étape a) et de manière préférée entre toute 6 heures après l'étape a).

Un autre aspect de l'invention se rapporte à une méthode d'évaluation de la force sensibilisante d'un composé test comprenant les étapes suivantes :
1) obtention d'au moins une dilution du composé test, et
2) détermination du potentiel sensibilisant dudit composé test à ladite au moins une dilution par une méthode selon la présente invention.

On entend par « force sensibilisante », la capacité d'un composé donné à induire une réponse sensibilisante en fonction de la concentration dudit composé. La force sensibilisante est dépendante de la quantité de substance nécessaire à l'induction de la sensibilisation. Ainsi, plus la quantité de sensibilisant nécessaire pour induire une réponse sensibilisante est faible, plus le sensibilisant est fort, et inversement, plus la quantité de sensibilisant nécessaire pour induire une réponse sensibilisante est élevée, plus le sensibilisant est faible.
Il est ainsi possible de réaliser une analyse quantitative du potentiel sensibilisant d'un composé.

De préférence, ledit composé test est soumis à des dilutions successives. Ainsi, les étapes 1) et 2) seront effectuées pour chacune des dilutions.

De préférence, lesdites dilutions successives permettront de déterminer la dilution maximale à laquelle ledit composé test conserve un potentiel sensibilisant.

Ladite méthode d'évaluation de la force sensibilisante d'un composé test peut en outre comprendre une étape d'évaluation de la force sensibilisante du composé test.

Ainsi, plus un produit conserve un potentiel sensibilisant suite à des dilutions successives, plus ledit composé test est un sensibilisant fort.

En outre, la force sensibilisante d'un composé test sera également fonction du potentiel irritant dudit composé test. Ainsi le fait qu'un composé test présente un potentiel irritant, augmente la force sensibilisante dudit composé test.

Par conséquent, dans un mode de réalisation préféré, ladite méthode comprend en outre une étape de détermination du potentiel irritant du composé test.

Le potentiel irritant d'un composé test peut par exemple être évalué en utilisant la méthode décrite dans la demande de brevet français n° 1051638.

Ainsi, on pourra conclure qu'un produit est extrêmement sensibilisant (Extrême E), si :
- celui-ci présente un potentiel sensibilisant à une dilution de 1/1000, et/ou
- celui-ci présente un potentiel sensibilisant à une dilution de 1/100, mais ne présente pas de potentiel sensibilisant à une dilution de 1/1000, et présente un potentiel irritant.

Ainsi, on pourra conclure qu'un produit est fortement sensibilisant (Fort (strong-S)), si :
- celui-ci présente un potentiel sensibilisant à une dilution de 1/100, mais ne présente pas de potentiel sensibilisant à une dilution de 1/1000, ou
- celui-ci présente un potentiel sensibilisant à une dilution de 1/10, mais ne présente pas de potentiel sensibilisant à une dilution de 1/100 et présente un potentiel irritant.

Ainsi, on pourra conclure qu'un produit est modérément sensibilisant (Modéré (Moderate M)), si :
- celui-ci présente un potentiel sensibilisant à une dilution de 1/10, mais ne présente pas de potentiel sensibilisant à une dilution de 1/100, ou
- celui-ci présente un potentiel sensibilisant à une dilution de 1/2, mais ne présente plus de potentiel sensibilisant à des dilutions inférieures à 1/2 et présente un potentiel irritant.

Ainsi, on pourra conclure qu'un produit est faiblement sensibilisant (faible (weak-W)), si celui-ci ne présente plus de potentiel sensibilisant à des dilutions inférieures à 1/2.

Selon un autre aspect, la présente invention se rapporte à l'utilisation du niveau d'expression dans un échantillon de peau reconstruite *in vitro*, d'au moins 6, au moins 7 ou au moins 8 gènes choisis dans le groupe constitué par : BRAK (CXC chimiokine ligand 14), CTSS(cathepsine S), DAPK2 (protéine kinase associée à la mort 2), FABP4 (Proteine de fixation des acides gras 4), HSP27 (Protéine de choc thermique de27kDa), IL18 (Interleukine 18), HSP90 (Protéine de choc thermique de 90kDa), IL1R2 (Recepteur type II de l'interleukine 1), TPSAB1 (tryptase alpha/beta 1), CXCR1 (Recepteur alpha de l'interleukin 8), DEFB1 (defensine, beta 1), DHFR (dihydrofolate reductase), EHF (Facteur homologue ets), IVL (involucrine), KRT4 (keratine 4), MELANA (melane-A), NGAL (Gelatinase des neutrophiles), PDZK11P1 (Protéine d'interaction PDZK1), PI3 (Inhibiteur de peptidase), PSME2 (Sous unité activatrice du proteasome 2), SERPINB3 (Membre 3 des Inhibiteurs de la serpine peptidase), AKR1B10 (Membre B10 de la famille des aldo-keto reductases), AKR1C1 (Membre C1 de la famille des aldo-keto reductases), AKR1C2 (Membre C2 de la famille des aldo-keto reductases), CYP1B1 (cytochrome P450, famille 1, sous famille B, polypeptide 1), FTH1P (Polypeptide lourd de la ferritine, 1), FTL (Polypeptide léger de la ferritine, 1), G6PD (glucose-6-phosphate dehydrogenase), GCLM (Sous unité modificatrice de la glutamate-cysteine ligase), NQO2 (NAD(P)H dehydrogenase des quinone s2), SLC7A11 (Membre 11 de la famille 7 des porteurs de molécules solubles), TXNRD1 (thioredoxine reductase 1), UGT1A1 (Polypeptide A1 de la famille des UDP glucuronosyltransferase 1), UGT1A9 (Polypeptide A9 de la famille des UDP glucuronosyltransferase 1 et YWHAZ (Polypeptide zeta de la protéine d'activation de tyrosine 3-monooxygenase/tryptophan 5-monooxygenase polypeptide) pour l'évaluation *in vitro* du potentiel sensibilisant d'un composé test.

La présente description divulgue également une trousse pour la mise en oeuvre d'une méthode d'évaluation *in vitro* du potentiel sensibilisant d'un composé test, comprenant des moyens de détermination du taux d'expression d'au moins six gènes choisis dans le groupe constitué par BRAK (CXC chimiokine ligand 14), CTSS (cathepsine S), DAPK2 (protéine kinase associée à la mort 2), FABP4 (Proteine de fixation des acides gras 4), HSP27 (Protéine de choc thermique de27kDa), IL18 (Interleukine 18), HSP90 (Protéine de choc thermique de 90kDa), IL1R2 (Recepteur type II de l'interleukine 1), TPSAB1 (tryptase alpha/beta 1), CXCR1 (Recepteur alpha de l'interleukin 8), DEFB1 (defensine, beta 1), DHFR (dihydrofolate reductase), EHF (Facteur homologue ets), IVL (involucrine), KRT4 (keratine 4), MELANA (melane-A), NGAL (Gelatinase des neutrophiles), PDZK11P1 (Protéine d'interaction PDZK1), PI3 (Inhibiteur de peptidase), PSME2 (Sous unité activatrice du proteasome 2), SERPINB3 (Membre 3 des Inhibiteurs de la serpine peptidase), AKR1B10 (Membre B10 de la famille des aldo-keto reductases), AKR1C1 (Membre C1 de la famille des aldo-keto reductases), AKR1C2 (Membre C2 de la famille des aldo-keto reductases), CYP1B1 (cytochrome P450, famille 1, sous famille B, polypeptide 1), FTH1P (Polypeptide lourd de la ferritine, 1), FTL (Polypeptide léger de la ferritine, 1), G6PD (glucose-6-phosphate dehydrogenase), GCLM (Sous unité modificatrice de la glutamate-cysteine ligase), NQO2 (NAD(P)H dehydrogenase des quinone s2), SLC7A11 (Membre 11 de la famille 7 des porteurs de molécules solubles), TXNRD1 (thioredoxine reductase 1), UGT1A1 (Polypeptide A1 de la famille des UDP glucuronosyltransferase 1), UGT1A9 (Polypeptide A9 de la famille des UDP glucuronosyltransferase 1, YWHAZ (Polypeptide zeta de la protéine d'activation de tyrosine 3-monooxygenase/tryptophan 5-monooxygenase polypeptide), CD36 (CD36 molecule), CYP1A1 (cytochrome P450, famille 1, sous famille A, polypeptide 1), GCLC (Sous unité catalitique de la glutamate-cysteine ligase), HMOX1 (heme oxygenase 1), NQO1 (NAD(P)H dehydrogenase,des quinones 1) et S100A8 (Protéine de fixation du calcium S100 A8).

La trousse ici décrite comprendra au moins six paires d'amorces permettant chacune l'amplification d'au moins un gène choisi dans le groupe constitué par : BRAK(CXC chimiokine ligand 14), CTSS (cathepsine S), DAPK2 (protéine kinase associée à la mort 2), FABP4 (Proteine de fixation des acides gras 4), HSP27 (Protéine de choc thermique de27kDa), IL18 (Interleukine 18 ), IL1R2 (Recepteur type II de l'interleukine 1), HSP90 (Protéine de choc thermique de 90kDa), TPSAB1 (tryptase alpha/beta 1), CXCR1 (Recepteur alpha de l'interleukin 8), DEFB1 (defensine, beta 1), DHFR (dihydrofolate reductase), EHF (Facteur homologue ets), IVL (involucrine), KRT4 (keratine 4), MELANA (melane-A), NGAL (Gelatinase des neutrophiles), PDZK11P1 (Protéine d'interaction PDZK1), PI3 (Inhibiteur de peptidase), PSME2 (Sous unité activatrice du proteasome 2), SERPINB3 (Membre 3 des Inhibiteurs de la serpine peptidase), AKR1B10 (Membre B10 de la famille des aldo-keto reductases), AKR1C1 (Membre C1 de la famille des aldo-keto reductases), AKR1C2 (Membre C2 de la famille des aldo-keto reductases), CYP1B1 (cytochrome P450, famille 1, sous famille B, polypeptide 1), FTH1P (Polypeptide lourd de la ferritine, 1), FTL (Polypeptide léger de la ferritine, 1), G6PD (glucose-6-phosphate dehydrogenase), GCLM (Sous unité modificatrice de la glutamate-cysteine ligase), NQO2 (NAD(P)H dehydrogenase des quinone s2), SLC7A11 (Membre 11 de la famille 7 des porteurs de molécules solubles), TXNRD1 (thioredoxine reductase 1), UGT1A1 (Polypeptide A1 de la famille des UDP glucuronosyltransferase 1), UGT1A9 (Polypeptide A9 de la famille des UDP glucuronosyltransferase 1, YWHAZ (Polypeptide zeta de la protéine d'activation de tyrosine 3-monooxygenase/tryptophan 5-monooxygenase polypeptide), CD36 (CD36 molecule), CYP1A1 (cytochrome P450, famille 1, sous famille A, polypeptide 1), GCLC (Sous unité catalitique de la glutamate-cysteine ligase), HMOX1 (heme oxygenase 1), NQO1 (NAD(P)H dehydrogenase,des quinones 1) et S100A8 (Protéine de fixation du calcium S100 A8).

De manière particulièrement préférée, ladite au moins une paire d'amorce est choisie dans le tableau 1.

### Exemples

### 1) Mise en évidence de biomarqueurs selon le protocole Skinethic

Les peaux, 1,07 cm², ont été achetées chez EPISKIN. Les différentes substances ont été appliquées soit sous forme liquide (30µl à différentes concentrations) soit sous forme solide (30µl PBS ou huile d'olive + 30µg ou moins pour évaluer différentes concentrations de poudre) sur les peaux. Après une incubation de 15mn à température ambiante les peaux ont été lavées avec du PBS (25 ml) et incubées 3h, 6h ou 18h à 37°C dans une étuve à CO₂. Après incubation les peaux ont été prélevées avec un punch et séparées du support en collagène. Elles ont ensuite été directement placées dans une solution de « Tri Reagent » (Ambion) (1 ml) et immédiatement dissociées mécaniquement.

### Préparation de l'ADNc

Les tissus ont été placés dans une solution de «Tri Reagent» (Ambion) et broyés mécaniquement. L'ARN a été préparé suivant le protocole décrit par le fournisseur avec une précipitation à l'isopropanol. Pour la préparation de l'ADNc, les ARN totaux ont été traités au préalable avec une DNAse pour retirer les ADN génomiques contaminants. On utilise pour le traitement 1 à 5 µg d'ARN total avec de la DNAse RNAse free, de la RNAsin (1 µl) et des random primers (3 µg). On ajoute ensuite de la superscipt III RT (1,5 µl à 200U/µl). Les cDNA sont ensuite testés par RT-PCR.

### PCR quantitative

La PCR quantitative en temps réel a été réalisée en utilisant la technologie SYBR Green des appareils LC480 de chez Roche. Les amorces ont été conçues pour couvrir les jonctions intron-exon pour prévenir d'éventuelles traces d'amplification de l'ADN génomique. L'amplification donne des amplicons entre 100 et 150 pb. Toutes les paires d'amorces ont été qualifiés par digestion avec des enzymes de restriction et analysés par électrophorèse. La PCR a été réalisée dans 10 µl en utilisant le mix PCR Sybr green 2X mix PCR de Roche dans des plaques PCR de chez Roche. L'expression des gènes cibles a été mesurée après normalisation de l'ARN grâce à quatre gènes de ménage, et les valeurs sont exprimées en utilisant la méthode des CT, et exprimés en taux d'expression supplémentaire par rapport à un zéro théorique (user bulletin no. 2, Applied Biosystems, December 1997).

### Résultants

Pour cette analyse, une application de 15mn suivi d'un lavage et d'une post incubation de 6h avant collecte des biopsies et d'une analyse de la transcription des gènes ont été réalisés. Les résultats sont présentés dans le tableau 1.

**Tableau 1 : taux d'expression des gènes spécifiques de la sensibilisation dans le modèle EPISKIN**

| **Abréviation** | **Nom du gène** | **Nom anglais** | **Numéro d'accès** | **Taux d'expression en l'absence de sensibilisant** | **Taux d'express ion en présence de sensibilis ant** | **Amorce sens** | **Amorce antisens** |
|---|---|---|---|---|---|---|---|
| **BRAK** | CXC chimiokine ligand 14 | chemokine (C-X-C motif) ligand 14 | NM_004887.4 | 0,653688171 | 1,8 | AAGTACCCGCACT GCGAGGAGAAG (SEQ ID NO : 1) | TTGGTGCTCTGCAGCTT GGGGTGC (SEQ ID NO : 2) |
| **HSP90** | Protéine de choc thermique de 90kDa | Heat shock 90kDa protein | NM_005348.3 | 1,054321721 | 1,75 | TCTGCCTCTGGTGA TGAGATGGTT(SEQ ID NO : 81) | TTTCCGAAGACGTTCC ACAAAGGC (SEQ ID NO : 82) |
| **CTSS** | cathepsine S | cathepsin S | NM_004079.3 | 0,51868364 | 2,296020 251 | TCATACGATCTGGG CATGAACCAC (SEQ ID NO : 3) | TGGGAACTCTCAGGGA ACTCATCA (SEQ ID NO : 4) |
| **DAPK2** | protéine kinase associée à la mort 2 | death-associated protein kinase 2 | NM_014326.3 | 1,012589915 | 1,454643 548 | TTTCCTTCAGCATC GTGTCCCTGT (SEQ ID NO : 5) | TGTCACTCTCACAGTTC CTCAGGT (SEQ ID NO : 6) |
| **FABP4** | Proteine de fixation des acides gras 4 | fatty acid binding protein 4, adipocyte | NM_001442.2 | 0,844425344 | 2,920748 581 | ACTGGGCCAGGAA TTTGACGAAGT (SEQ ID NO : 7) | TTTCTGCACATGTACCA GGACACC (SEQ ID NO : 8) |
| **HSP27** | Protéine de choc thermique de27kDa | heat shock 27kDa protein 1 | NM_001540.3 | 0,868543127 | 1,612936 908 | TGCTTCACGCGGAA ATACACGCT (SEQ ID NO : 9) | CTGGGATGGTGATCTC GTTGGACT (SEQ ID NO :10) |
| **IL18** | Interleukine 18 | Interleukin 18 (interferon-gamma-inducing factor) | NM_001562.2 | 1,190956418 | 2,309301 387 | TCATTGACCAAGG AAATCGGCCTC (SEQ ID NO :11) | AGCCATACCTCTAGGC TGGCTAT (SEQ ID NO : 12) |
| **IL1R2** | Recepteur type II de l'interleukine 1 | interleukin 1 receptor, type II | NM_173343.1 | 1,280621228 | 1,9 | GCCAGCCTTGCAG GAGGACTCTG (SEQ ID NO : 13) | TTGCGGGTATGAGATG AACGGCAG (SEQ ID NO : 14) |
| **TPSAB1** | tryptase alpha/beta 1 | tryptase alpha/beta 1 | NM_003294.3 | 0,048878149 | 33,92776 528 | ACGGCCCATACTG GATGCACTTCT (SEQ ID NO : 15) | CAGCAGCTGGTCCTGG TAGTAGA (SEQ ID NO : 16) |
| **CXCR1** | Récepteur alpha de l'interleukin 8 | interleukin 8 receptor, alpha | NM_000634.2 | 1,442894074 | 3,492178 966 | TATGAATCTGTCCC TGCCCTTC (SEQ ID NO : 17) | ACCTCATAGCAAACTG GACTGGAA (SEQ ID NO : 18) |
| **DEFB1** | defensine, beta 1 | defensin, beta 1 | NM_005218.3 | 0,667424597 | 2,431463 704 | TTCCTGAAATCCTG GGTGTTGCCT (SEQ ID NO : 19) | AGGCCTGTGAGAAAGT TACCACCT (SEQ ID NO : 20) |
| **DHFR** | dihydrofolate reductase | dihydrofolate reductase | NM_000791.3 | 1,033017625 | 2,6 | CTCATTTTCTTTCC AGAAGTCTAG (SEQ ID NO 21) | TGCCACCAACTATCCA GACCATGT (SEQ ID NO : 22) |
| **EHF** | Facteur homologue ets | ets homologous factor | NM_012153.3 | 0,827499527 | 2,815783 657 | TTGGCTCTCTCATG TCCTTGGCTT (SEQ ID NO : 23) | AGGTATGACACTGTGG TAGGTGCT (SEQ ID NO : 24) |
| **IVL** | involucrine | involucrin | NM_005547.2 | 0,943876521 | 1,859505 427 | TGCCCACAAAGGG AGAAGTATTGC (SEQ ID NO : 25) | TCTGGACACTGCGGGT GGTTATTT (SEQ ID NO : 26) |
| **KRT4** | keratine 4 | keratin 4 | NM_002272.2 | 0,925148858 | 1,357420 71 | TCACATATGTCCCT TCCCAGTCCA (SEQ ID NO : 27) | TGCCGGGTGTTGGAGA AGTAGTTT (SEQ ID NO : 28) |
| **MELANA** | melane-A | melan-A | NM_005511.1 | 0,763327598 | 2,7 | TCTTACTGCTCATC GGCTGTTGGT (SEQ ID NO : 29) | TGAAGAGACACTTTGC TGTCCCGA (SEQ ID NO : 30) |
| **NGAL** | Gelatinase des neutrophiles | lipocalin 2 | NM_005564.3 | 1,069357565 | 1,8 | GTGAGCACCAACT ACAACCAGCAT (SEQ ID NO : 31) | AGTTCCGAAGTCAGCT CCTTGGTT (SEQ ID NO : 32) |
| **PDZK11P1** | Protéine d'interaction PDZK1 | PDZK1 interacting protein 1 | NM_005764.3 | 0,705479404 | 2,3 | CAATCGCCTTTGCA GTCAACCACT (SEQ ID NO : 33) | ACCAGGACTCCATCTG CCTTGTTT (SEQ ID NO : 34) |
| **PI3** | Inhibiteur de peptidase | peptidase inhibitor 3, skin-derived | NM_002638.3 | 1,19471567 | 2,5 | TCTTGATCGTGGTG GTGTTCCTCA (SEQ ID NO : 35) | GACTGGCTCTTGCGCTT TGACTTT (SEQ ID NO : 36) |
| **PSME2** | Sous unité activatrice du proteasome 2 | proteasome activator subunit 2 (PA28 beta) | NM_002818.2 | 1,066067422 | 1,9 | TCCCTCAATGTGGC TGACTTGACT (SEQ ID NO : 37) | TCTCATTCCCAGGGAG AAATCCAC (SEQ ID NO : 38) |
| **SERPINB3** | Membre 3 des Inhibiteurs de la serpine peptidase | serpin peptidase inhibitor, clade B (ovalbumin), member 3 | NM_006919.2 | 0,462228737 | 4,366336 795 | ACTCCTGGGTGGA AAGTCAAACGA (SEQ ID NO : 39) | ACTCCTGGGTGGAAAG TCAAACGA (SEQ ID NO : 40) |
| **AKR1B10** | Membre B10 de la famille des aldo-keto reductases | aldo-keto reductase family 1, member B10 (aldose reductase) | NM_020299.4 | 1,219844195 | 1,9 | AAGATGATAAAGG TAATGCCATCG (SEQ ID NO : 41) | AGCTTCTCGATCTGGA AGTGGCTG (SEQ ID NO : 42) |
| **AKR1C1** | Membre C1 de la famille des aldo-keto reductases | aldo-keto reductase family 1, member C1 | NM_001353.5 | 1,587681403 | 3,432182 344 | GCAGAGGTTCCTA AAAGTAAAGCTTT A (SEQ ID NO : 43) | ACCTGCTCCTCATTATT GTATAAATGA (SEQ ID NO : 44) |
| **AKR1C2** | Membre C2 de la famille des aldo-keto reductases | aldo-keto reductase family 1, member C2 | NM_001354.4 | 0,686185206 | 3,101161 617 | CATTGCATGAGGTC TGCCAGAAGG (SEQ ID NO : 45) | CTTAGCTGTAGCTTACT GAAGTCG (SEQ ID NO : 46) |
| **CYP1B1** | cytochrome P450, famille 1, sous famille B, polypeptide 1 | cytochrome P450, family 1, subfamily B, polypeptide 1 | NM_000104.3 | 0,751299588 | 17,37117 803 | ATCAACAAGGACC TGACCAGCAGA (SEQ ID NO : 47) | TCATTTGGGTTGGCCCT GAAATCG (SEQ ID NO : 48) |
| **FTH1P** | Polypeptide lourd de la ferritine, 1 | ferritin, heavy polypeptide 1 | NM_002032.2 | 1,032878604 | 1,942318 842 | CTTTGACCGCGATG ATGTGGCTTT (SEQ ID NO : 49) | TCAGTTTCTCAGCATGT TCCCTCT (SEQ ID NO : 50) |
| **FTL** | Polypeptide léger de la ferritine, 1 | ferritin, light polypeptide | NM_000146.3 | 0,681444874 | 2,940199 851 | TTGGATCTTCATGC CCTGGGTTCT (SEQ ID NO : 51) | AGTCGTGCTTGAGAGT GAGCCTTT (SEQ ID NO : 52) |
| **G6PD** | glucose-6-phosphate dehydrogenase | glucose-6-phosphate dehydrogenase | NM_000402.3 | 0,581030262 | 1,7 | GAACCTCATGGTGC TGAGATTTGC (SEQ ID NO : 53) | TGAGGATAACGCAGGC GATGTTGT (SEQ ID NO : 54) |
| **GCLM** | Sous unité modificatrice de la glutamate-cysteine ligase | glutamate-cysteine ligase, modifier subunit | NM_002061.2 | 0,874803304 | 5,083406 994 | ATGGCCTGTTCAGT CCTTGGAGTT (SEQ ID NO : 55) | TCCCAGTAAGGCTGTA AATGCTCC (SEQ ID NO : 56) |
| **NQO2** | NAD(P)H dehydrogenase des quinone s2 | NAD(P)H dehydrogenase, quinone 2 | NM_000904.3 | 1,170224823 | 2,8 | CACGAAGACAGGA GTCAATGGAGA (SEQ ID NO : 57) | CGGATGCAATTTCAGG AGCAAAGC (SEQ ID NO : 58 ) |
| **SLC7A11** | Membre 11 de la famille 7 des porteurs de molécules solubles | solute carrier family 7, (cationic amino acid transporter, y+ system) member 11 | NM_014331.3 | 0,502347786 | 5,088930 603 | TATCCCTGGCATTT GGACGCTACA (SEQ ID NO : 59) | TGCCCACAGCTGTAAT GAGCTTGA (SEQ ID NO : 60) |
| **TXNRD1** | thioredoxine reductase 1 | thioredoxin reductase 1 | NM_182729.1 | 0,725407592 | 3,707572 92 | GGTCCTCACAGGAT TAAGGCAACA (SEQ ID NO : 61) | TGCCCAAGTAACGTGG TCTTTCAC (SEQ ID NO : 62) |
| **UGT1A1** | Polypeptide A1 de la famille des UDP glucuronosyltransferase 1 | UDP glucuronosyltransferas e 1 family, polypeptide A1 | NM_000463.2 | 0,527392251 | 1,408908 304 | ACAGAACTTTCTGT GCGACGTGGT (SEQ ID NO : 63) | AGCCAGACAGATGCAG AGCTCAAT (SEQ ID NO : 64) |
| **UGT1A9** | Polypeptide A9 de la famille des UDP glucuronosyltransferase 1 | UDP glucuronosyltransferas e 1 family, polypeptide A9 | NM_021027.2 | 0,568212175 | 1,402794 916 | GCTTTGCCGAGGCA GGGAAGCTAC (SEQ ID NO : 65) | ATGAGTTTCTCCACCA CCGACCTC (SEQ ID NO : 66) |
| **YWHAZ** | Polypeptide zeta de la protéine d'activation de tyrosine 3-monooxygenase/tryptophan 5-monooxygenase polypeptide | tyrosine 3-monooxygenase/trypto phan 5-monooxygenase activation protein, zeta polypeptide | NM_001135702. 1 | 1,154023086 | 3,1 | ACAGCAGATGGCT CGAGAATACAG (SEQ ID NO : 67) | TGCTCTCTGCTTGTGAA GCATTGG (SEQ ID NO : 68) |
| **CD36** | CD36 molecule | CD36 molecule (thrombospondin receptor) | NM_001127444. 1 | 0,640243459 | 5,375531 509 | ACAGATGCAGCCT CATTTCCACCT (SEQ ID NO : 69) | GGGATTCCTTTCAGATT AACGTCGG (SEQ ID NO : 70) |
| **CYP1A1** | cytochrome P450, famille 1, sous famille A, polypeptide 1 | cytochrome P450, family 1, subfamily A, polypeptide 1 | NM_000499.3 | 0,549457486 | 3,763056 606 | AGTGGCAGATCAA CCATGACCAGA (SEQ ID NO : 71) | ACACCTTGTCGATAGC ACCATCAG (SEQ ID NO : 72) |
| **GCLC** | Sous unité catalitique de la glutamate-cysteine ligase | glutamate-cysteine ligase, catalytic subunit | NM_001498.2 | 0,905634705 | 2,728685 624 | AATGGGCAATTGCT GTCTCCAGGT (SEQ ID NO : 73) | AAAGGAGATGCAGCA CTCAAAGC (SEQ ID NO : 74) |
| **HMOX1** | heme oxygenase 1 | heme oxygenase (decycling) 1 | NM_002133.1 | 0,76137536 | 1,461320 376 | GGGCCAGCAACAA AGTGCAAGATT (SEQ ID NO : 75) | TCGCCACCAGAAAGCT GAGTGTAA (SEQ ID NO : 76) |
| **NQO1** | NAD(P)H dehydrogenase,des quinones 1 | NAD(P)H dehydrogenase, quinone 1 | NM_001025434. 1 | 0,740574662 | 1,948391 073 | AGTGGCTCCATGTA CTCTCTGCAA (SEQ ID NO : 77) | TTCTCCAGGCGTTTCTT CCATCCT (SEQ ID NO : 78) |
| **S100A8** | Protéine de fixation du calcium S100 A8 | S100 calcium binding protein A8 | NM_002964.3 | 0,970410479 | 1,890390 064 | GGGATGACCTGAA GAAATTGCTA (SEQ ID NO : 79) | TGTTGATATCCAACTCT TTGAACCA (SEQ ID NO : 80) |

Par ailleurs, 35 substances classées selon leurs caractéristiques (non irritante (non IRR) non sensibilisante (NS), irritante (IRR) ou sensibilisante en utilisant la classification officielle en Extrême (E), Fort « strong, S », Intermédiaire, (intermediate M) et faible (weak-W) ont été testées à différentes doses (Tableau 2). Pour l'analyse, la dose permettant la réponse maximale sans induire de destruction tissulaire (corrosion) trop importante a été retenue.

| Classe | Nom | CAS# |
|---|---|---|
| S | Hydroquinone | 123-31-9 |
| S | 2-aminophenol | 95-55-6 |
| E | 4-Nitrobenzylbromide (electrophile) | 100-11-8 |
| S | 2,4,6-Trinitrobenzene sulfonic acid | 2508-19-2 |
| S | p-Phenylenediamine | 106-50-3 |
| M | Citral | 5392-40-5 |
| S | Dinitrochlorobenzene | 97-00-7 |
| M | Benzaldehyde | 100-52-7 |
| W | Resorcinol | 108-46-3 |
| M | Benzisothiazolin-3-one (Benzisothiazolinone) | 2634-33-5 |
| M | Disulfur TétraMéthylThiurame | 137-26-8 |
| S | Oxazolone | 15646-46-5 |
| S | ChloroAtranol | 57074-21-2 |
| E | Diphenylcyclopropénone | 886-38-4 |
| S | Potassium Dichromate | 7778-50-9 |
| M | aldéhyde cinnamique | 104-55-2 |
| S | 2-Bromo-2-(bromomethyl)glutaronitrile | 35691-65-7 |
| M | Glyoxal | 107-22-2 |
| M | Saccharine | 6485-34-3 |
| S | Formaldehyde | 50-00-0 |
| M | Trimellitic anhydride | 552-30-7 |
| E | MethylChlorolsothiazolinone | 26172-55-4 |
| M | Phenyl Acetaldehyde | 122-78-1 |
| W | Benzyl benzoate | 120-51-4 |
| W | Lilial | 80-5406 |
| W | alpha-Hexyl CinnAmaldehyde | 101-86-0 |
| W | Eugenol | 97-53-0 |
| M | 2mercaptobenzothiazole | 149-30-4 |
| S | Glutaraldehyde | 111-30-8 |

| | | |
|---|---|---|
| M | Isoeugenol | 97-54-1 |
| NS | Dipropylene Glycol | 25265-71-8 |
| NS | Glycerol | 56-81-5 |
| NS | Cetyl trimethylammonium bromide | 57-09-0 |
| NS | Limonen | 5989-54-8 |
| IRR | Acide Lactique (pos cys) | 598-82-3 |
| IRR | Sodium Lauryl Sulfate | 151-21-3 |

3 groupes de biomarqueurs ont été testés sur les échantillons, ceux spécifiques de l'irritation, ceux représentés par les gènes de la famille ARE (gènes sous le contrôle des promoteurs « ARE-antioxydant responsive élément », à savoir les gènes AKR1B10, AKR1C1, AKR1C2, CYP1B1, FTH1P, FTL, G6PD, GCLM, NQO2, SLC7A11, TXNRD1, UGT1A1, UGT1A9, YWHAZ, CD36, CYP1A1, GCLC, HMOX1, NQO1, PSME2 et S100A8) et un autre groupe de gènes spécifiques de la sensibilisation notamment pour les substances sensibilisantes qui n'induisent pas les gènes ARE.

Les irritants dont l'expression du gène IL-8 est 50 fois supérieure au contrôle et qui induisent une expression non spécifique des gènes ARE ont été retestés à des doses plus faibles.

Les résultats sont présentés dans les tableaux 2 et 3, avec un code selon le taux de surexpression des gènes. Si l'expression est supérieure à 1,3 par rapport au contrôle une note de 1 est donnée, si cette expression est inférieure, la note est 0. On constate que, de préférence, si le nombre de gènes ARE est supérieur à 11, alors on peut conclure que la substance est considérée de manière certaine comme sensibilisante. Pour les substances n'induisant pas fortement les gènes ARE, on analyse alors l'autre groupe de gènes de sensibilisation.
On observe que, de préférence, si au moins 8 de ces gènes sont surexprimés alors la substance est clairement sensibilisante.
De préférence, ledit test est réalisé à la fois sur le groupe des gènes ARE et le groupe des gènes « non-ARE ».

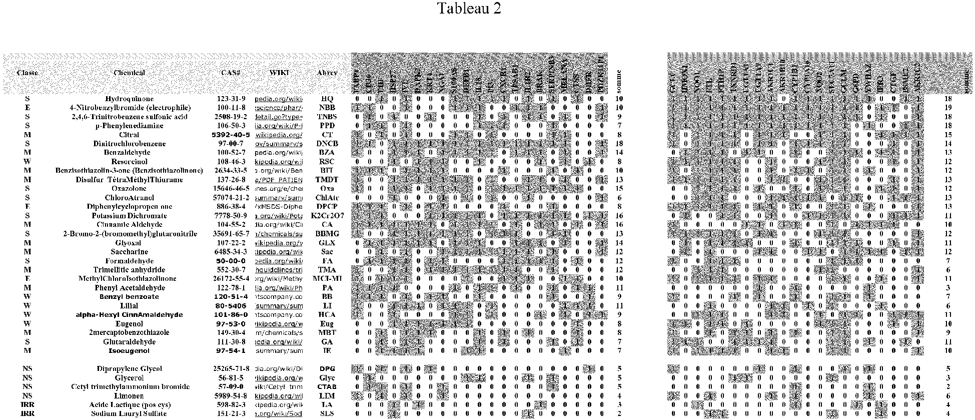

D'autre part, afin d'évaluer la force sensibilisante des composés, une sélection de composés a été soumise à des dilutions successives avant d'être à nouveau testés pour leur potentiel sensibilisant, comme le montre le tableau 3.

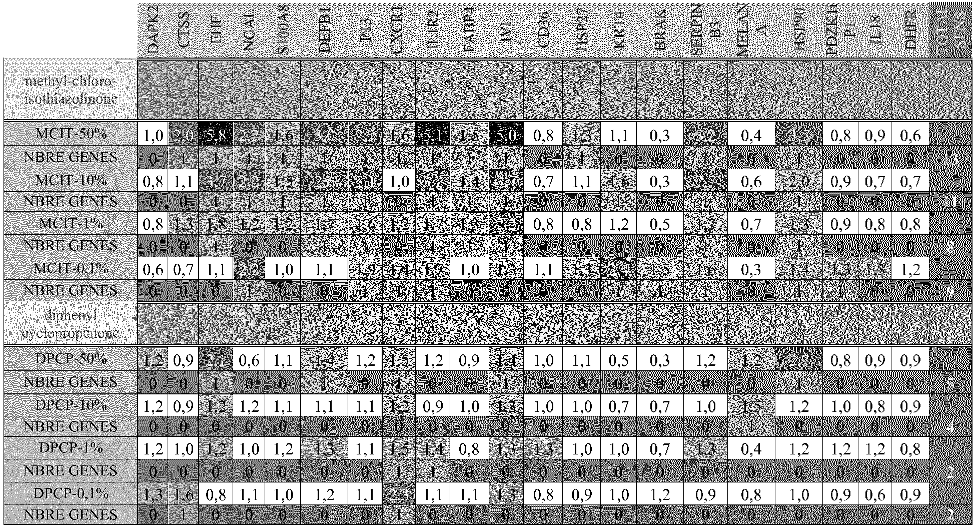

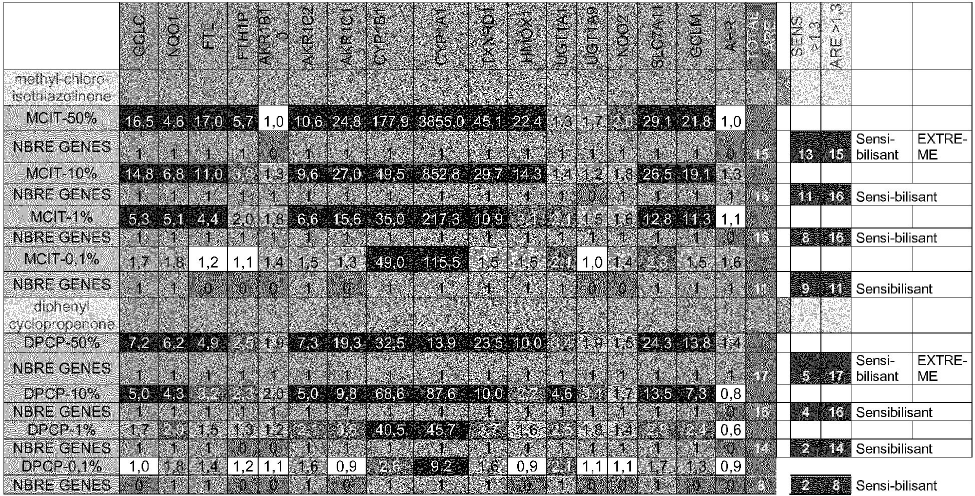

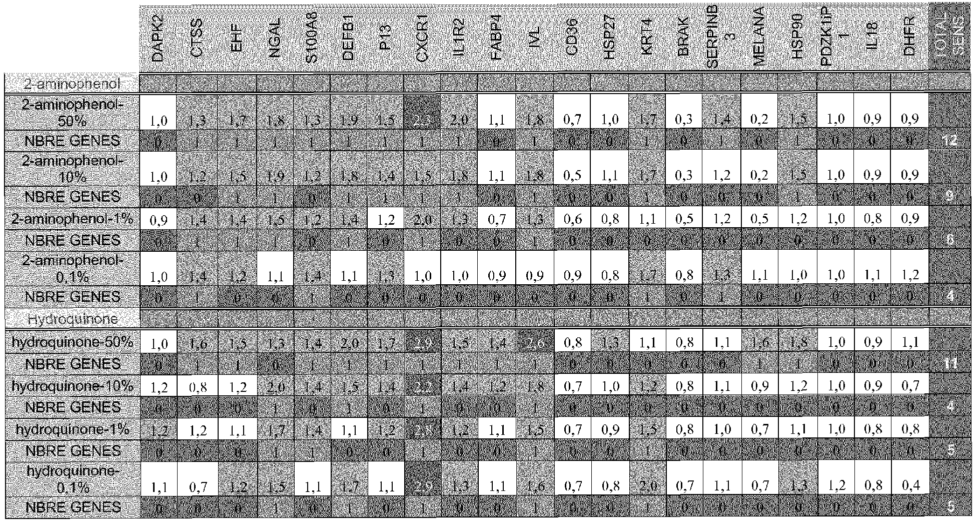

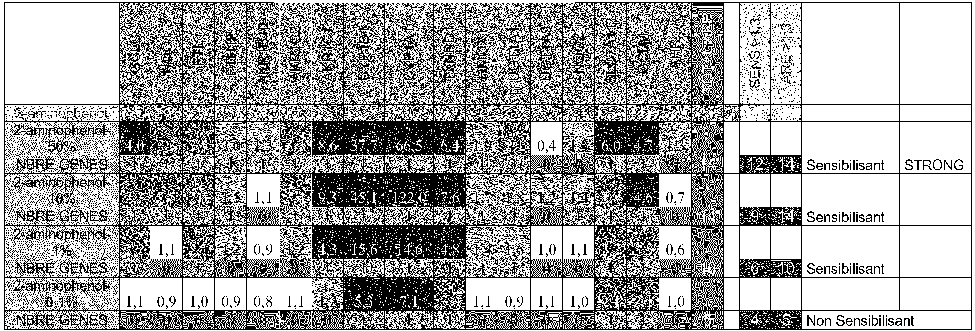

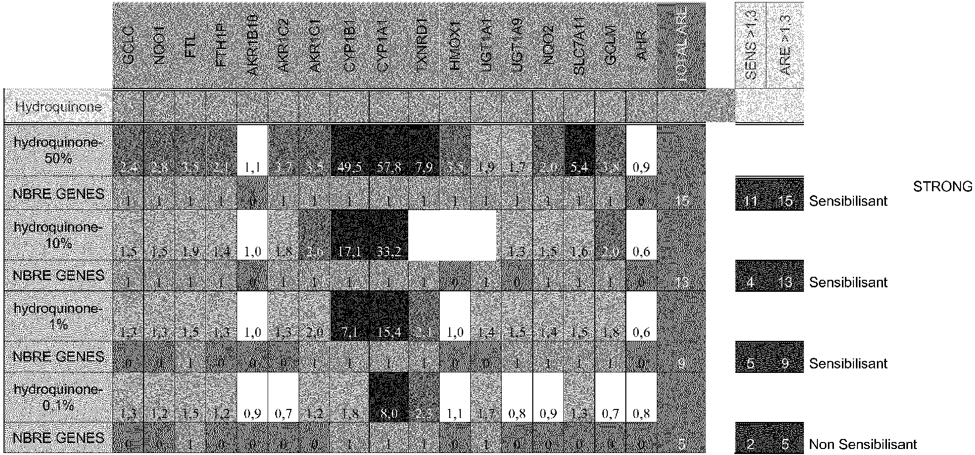

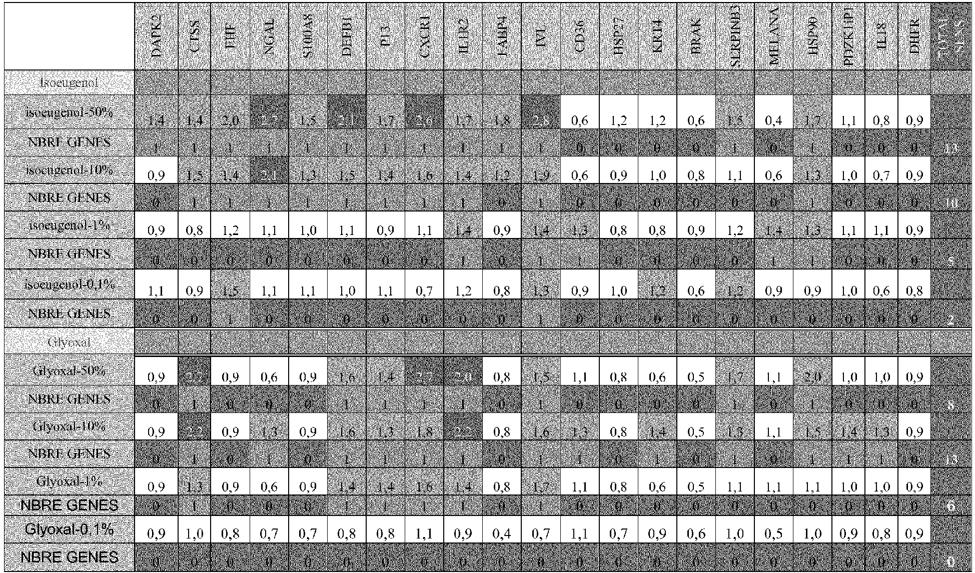

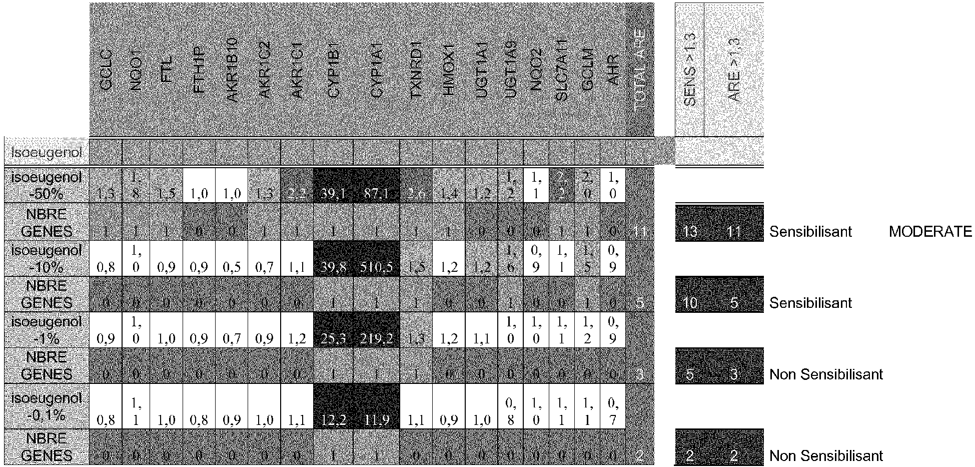

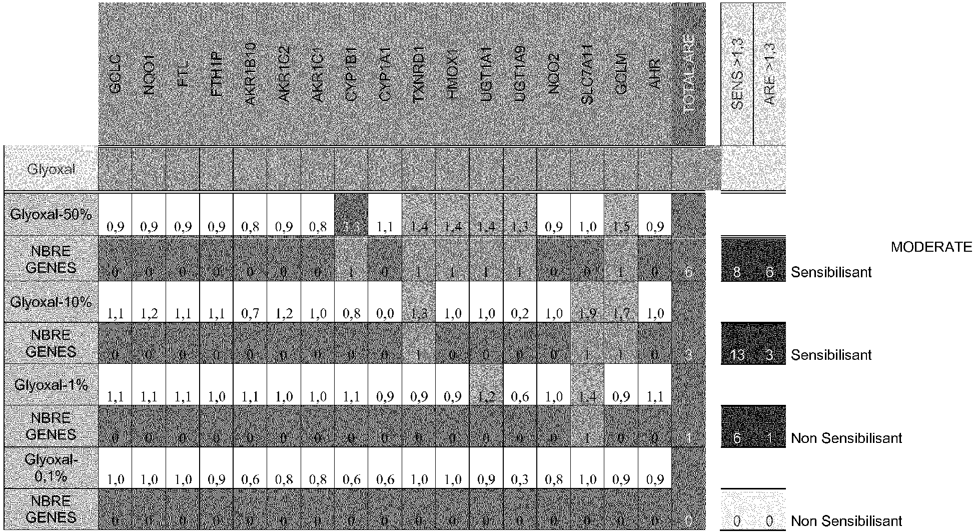

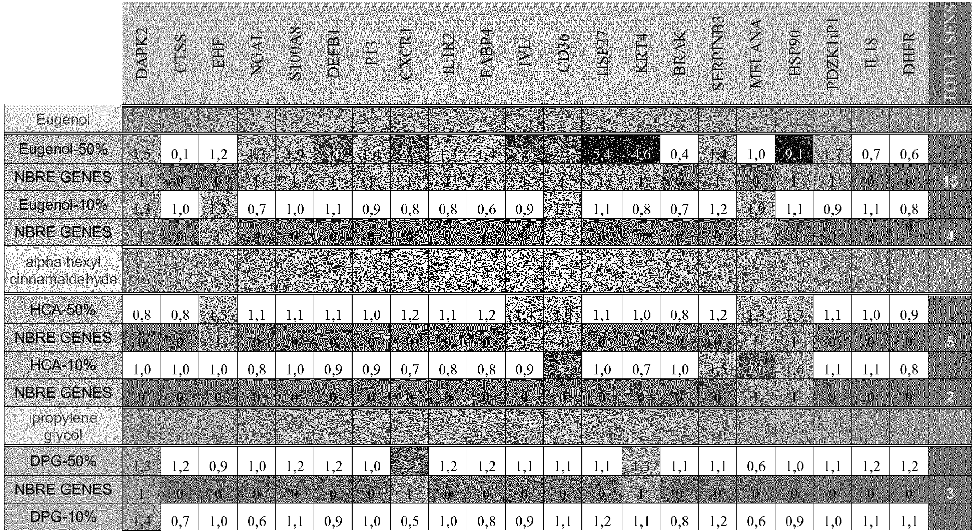

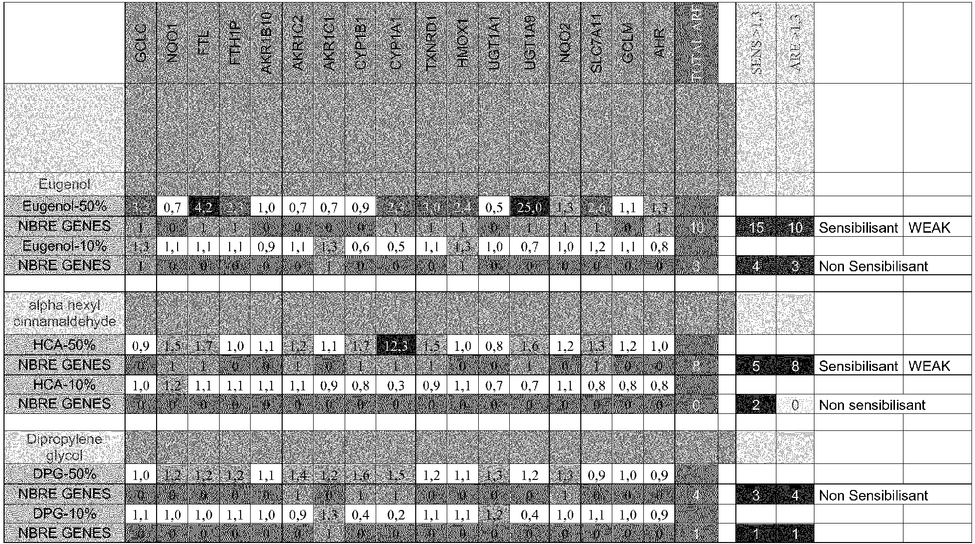

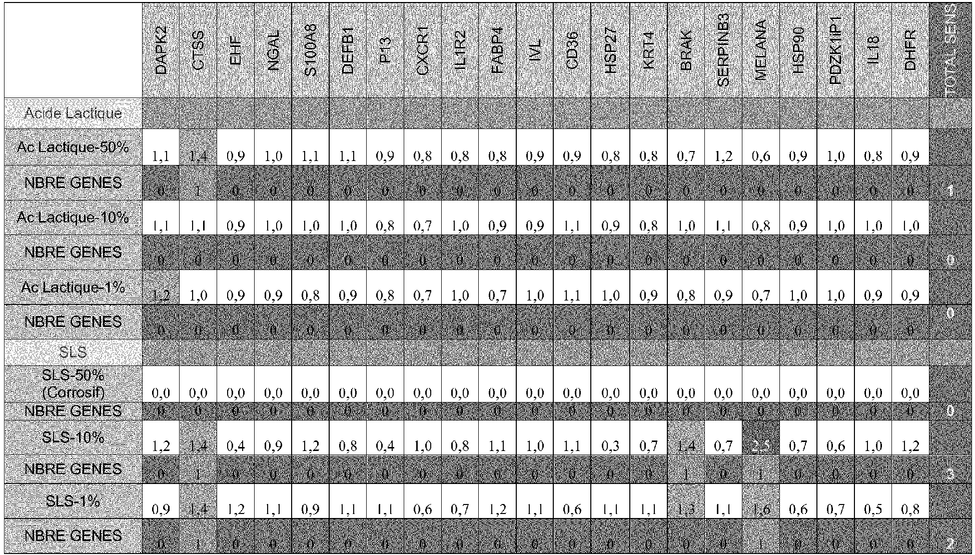

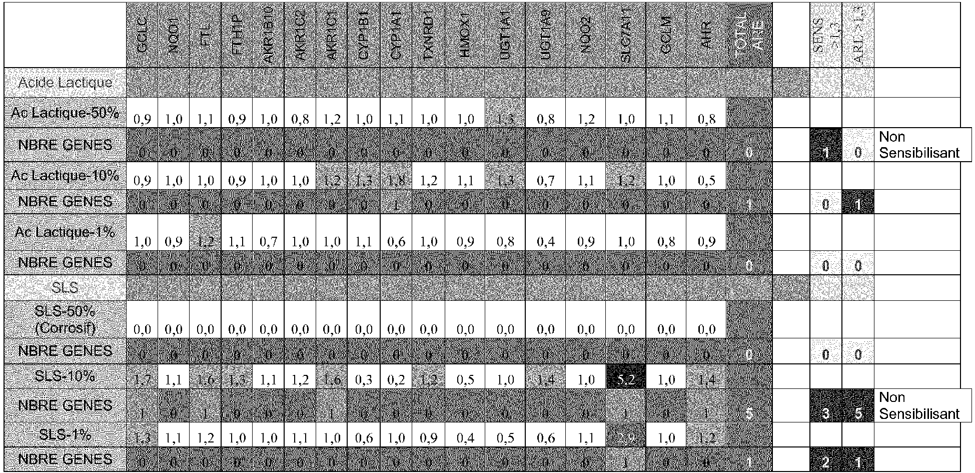

## Revendications

1. Méthode d'évaluation du potentiel sensibilisant d'un composé test, comprenant les étapes de:
a) mise en contact d'un composé test avec un échantillon de peau
b) détermination du niveau d'expression d'au moins six gènes choisis dans le groupe constitué par : BRAK (CXC chimiokine ligand 14), CTSS (cathepsine S), DAPK2 (protéine kinase associée à la mort 2), FABP4 (Protéine de fixation des acides gras 4), HSP27 (Protéine de choc thermique de27kDa), IL18 (Interleukine 18 ), HSP90 (Protéine de choc thermique de 90kDa), IL1R2 (Récepteur type II de l'interleukine 1), TPSAB1 (tryptase alpha/beta 1), CXCR1 (Recepteur alpha de l'interleukin 8), DFFB1 (defensine, beta 1), DHFR (dihydrofolate reductase), EHF (Facteur homologue ets), IVL (involucrine), KRT4 (kératine 4), MELANA (melane-A), NGAL (Gelatinase des neutrophiles), PDZK11P1 (Protéine d'interaction PDZK1), PI3 (Inhibiteur de peptidase), PSME2 (Sous unité activatrice du proteasome 2), SERPINB3 (Membre 3 des Inhibiteurs de la serpine peptidase), AKR1B10 (Membre B10 de la famille des aldo-keto reductases), AKR1C1 (Membre C1 de la famille des aldo-keto reductases). AKR1C2 (Membre C2 de la famille des aldo-keto reductases), CYP1B1 (cytochrome P450, famille 1, sous famille B, polypeptide 1), FTH1P (Polypeptide lourd de la ferritine, 1), FTL (Polypeptide léger de la ferritine, 1), G6PD (glucose-6-phosphate dehydrogenase), GCLM (Sous unité modificatrice de la glutamate-cysteine ligase), NQO2 (NAD(P)H dehydrogenase des quinone s2), SLC7A11 (Membre 11 de la famille 7 des porteurs de molécules solubles). TXNRD1 (thioredoxine reductase 1), UGT1A1 (Polypeptide A1 de la famille des UDP glucuronosyltransferase 1), UGT1A9 (Polypeptide A9 de la famille des UDP glucuronosyltransferase 1, YWHAZ (Polypeptide zeta de la protéine d'activation de tyrosine 3-monooxygenase/tryptophan 5-monooxygenase polypeptide), CD36 (CD36 molecule), CYP1A1 (cytochrome P450, famille 1, sous famille A, polypeptide 1), GCLC (Sous unité catalitique de la glutamate-cysteine ligase), HMOX1 (hernie oxygenase 1), NQO1 (NAD(P)H dehydrogenase,des quinoncs 1) et S100A8 (Protéine de fixation du calcium S100 A8), **caractérisée en ce que** l'échantillon de peau est un échantillon de peau reconstruite *in vitro*, et
c) détermination dudit composé comme présentant un potentiel sensibilisant si le niveau d'expression d'au moins six desdits gènes est supérieur à une valeur seuil.

2. Methode d'évaluation selon la revendication 1, dans laquelle le niveau d'expression de chacun desdits au moins six gènes est déterminé par mesure du niveau d'expression du polypeptide codé par ledit gene ou un fragment de celui-ci, ou par mesure du niveau d'expression de l'ARNm dudit au moins un gène ou d'un fragment de celui-ci.

3. Méthode d'évaluation selon l'une des revendications 1 ou 2, comprenant une étape supplémentaire d'amplification de l'ARNm ou de l'ADNc de chacun desdits au moins six gènes, de la séquence complémentaire de celle-ci ou d'un fragment de celle-ci.

4. Méthode selon l'une quelconque des revendications 1 à 3, dans laquelle l'étape b) comprend la mesure de l'expression d'au moins 7, d'au moins 8, d'au moins 9, d'au moins 10, d'au moins 11, d'au moins 12, d'au moins 13, d'au moins 14, d'au moins 15, d'au moins 16, d'au moins 17, d'au moins 18, d'au moins 19, d'au moins 20, d'au moins 21, d'au moins 22, d'au moins 23, d'au moins 24 et encore préférentiellement d'au moins 25 gènes choisis dans le groupe constitué par : BRAK (CXC chimiokine ligand 14), CTSS (cathepsine S), DAPK22 (protéine kinase associée à la mort 2), FABP4 (Protéine de fixation des acides gras 4), HSP27 (Protéine de choc athermique de27kDa), IL18 (Interleukine 18 ), HSP90 (Protéine de choc thermique de 90ka), IL1R2 (Recepteur type II de l'interleukine 1), TPSAB1 (tryptase alpha/beta 1), CXCR1 (Récepteur alpha de l'interleukin 8), DEFB1 (defensine, beta 1), DHFR (dihydrofolate reductase), EHF (Facteur homologue ets). IVL (involucrine), KRT4 (kératine 4). MELANA (melane-A), NGAL (Gclatinasc des neutrophiles), PDZK11P1 (Protéine d'interaction PDZK1). PI3 (Inhibiteur de peptidase), PSME2 (Sous unité activatrice du proteasome 2), SERPINB33 (Membre 3 des Inhibiteurs de la serpine peptidase), AKR1B10 (Membre B10 de la famille des aldo-keto reductases), AKRIC1 (Membre C1 de la famille des aldo-keto reductases), AKR1C2 (Membre C2 de la famille des aldo-keto reductases), CYP1B1 (cytochrome P450, famille 1, sous famille B, polypeptide 1), FTH1P (Polypeptide lourd de la ferritine, 1), FTL (Polypeptide léger de la ferritine, 1), G6PD (glucose-6-phosphate dehydrogenase), GCLM (Sous unité modificatrice de la glutamate-cysteine ligase), NQO2 (NAD(P)H dehydrogenase des quinone s2), SLC7A11 (Membre 11 de la famille 7 des porteurs de molécules solubles), TXNRD1 (thioredoxine reductase 1), UGT1A1 (Polypeptide A1 de la famille des UDP glucuronosyltransferase 1), UGT1A9 (Polypeptide A9 de la famille des UDP glucuronosyltransferase 1, YWHAZ (Polypeptide zeta de la protéine d'activation de tyrosine 3-monooxygenase/tryptophan 5-monooxygenase polypeptide), CD36 (CD36 molecule), CYP1A1 (cytochrome P450, famille 1, sous famille A, polypeptide 1), GCLC (Sous unité catalitique de la glutamate-cysteine ligase), HMOX1 (heme oxygenase 1), NQO1 (NAD(P)H dehydrogenase,des quinones 1) et S100A8 (Protéine de fixation du calcium S100 A8).

5. Méthode selon l'une quelconque des revendications précédentes, dans laquelle l'étape b) est réalisée entre 2 et 24 heures après l'étape a), de manière encore préférée entre 4 et 18 heures après l'étape a), de manière particulièrement préférée entre 5 et 7 heures après l'étape a) et de manière préférée entre toute 6 heures après l'étape a).

6. Méthode d'évaluation de la force sensibilisante d'un composé test comprenant les étapes suivantes :
1) obtention d'au moins une dilution du composé test, et
2) mesure du potentiel sensibilisant dudit composé test à ladite au moins une dilution par une méthode telle que définie selon l'une quelconque des revendications précédentes.

7. Utilisation du niveau d'expression, dans un échantillon de peau reconstruite *in vitro*, d'au moins 6 gènes choisis dans le groupe constitué par : BRAK(CXC chimiokine ligand 14), CTSS (cathepsine S), DAPK2 (protéine kinase associée à la mort 2), FABP4 (Protéine de fixation des acides gras 4), HSP27 (Protéine de choc thermique de27kDa), IL18 (Interleukine 18), HSP90 (Protéine de choc thermique de 90kDa), IL1R2 (Récepteur type II de l'interleukine 1), TPSAB1 (tryptase alpha/beta 1), CXCR1 (Recepteur alpha de l'interleukin 8), DEFB1 (defensine, beta 1), DHFR (dihydrofolate reductase), EHF (Facteur homologue ets), IVL (involucrine), KRT4 (keratine 4), MELANA (melane-A), NGAL (Gelatinase des neutrophiles), PDZK11P1 (Protéine d'interaction PDZK1), PI3 (Inhibiteur de peptidase), PSME2 (Sous unité activatrice du proteasome 2), SERPINB3 (Membre 3 des Inhibiteurs de la serpine peptidase), AKR1B10 (Membre B10 de la famille des aldo-keto reductases), AKR1C1 (Membre C1 de la famille des aldo-keto reductases), AKR1C2 (Membre C2 de la famille des aldo-keto reductases), CYP1B1 (cytochrome P450, famille 1, sous famille B, polypeptide 1), FTH1P (Polypeptide lourd de la ferritine, t), FTL (Polypeptide léger de la ferritine, 1), G6PD (glucose-6-phosphate dehydrogenase), GCLM (Sous unité modificatrice de la glutamate-cysteine ligase), NQO2 (NAD(P)H dehydrogenase des quinone s2), SLC7A11 (Membre 11 de la famille 7 des porteurs de molécules solubles), TXNRD1 (thioredoxine reductase 1), UGT1A1 (Polypeptide A1 de la famille des UDP glucuronosyltransferase 1), UGT1A9 (Polypeptide A9 de la famille des UDP glucuronosyltransferase 1 et YWHAZ (Polypeptide zeta de la protéine d'activation de tyrosine 3-monooxygcnasc/tryptophan 5-monooxygenase polypeptide) pour l'évaluation *in vitro* du potentiel sensibilisant d'un composé test.

## Patentansprüche

1. Methode zur Bewertung des Sensibilisationspotentials einer Testverbindung, die die folgenden Schritte umfasst:
a) Inkontaktbringen einer Testverbindung mit einer Hautprobe
b) Bestimmung des Expressionsspiegels von mindestens sechs Genen, die aus der sich folgendermaßen zusammensetzenden Gruppe ausgewählt werden: BRAK (CXC-Chemokinligand 14), CTSS (Kathepsin S), DAPK2 (zelltodassoziierte Proteinkinase 2), FABP4 (Fettsäuren bindendes Protein 4), HSP27 (Hitzeschockprotein von 27kDa), IL18 (Interleukin 18), HSP90 (Hitzeschockprotein von 90kDa), IL1R2 (Rezeptor Typ II des Interleukin 1), TPSAB1 (Alpha-/Beta-Tryptase 1), CXCR1 (Alpha-Rezeptor des Interleukin 8), DEFB1 (Beta-Defensin 1), DHFR (Dihydrofolatreduktase), EHF (homologer Faktor ETS), IVL (Involucrin), KRT4 (Keratin 4), MELANA (Melan-A), NGAL (Gelatinase der Neutrophile), PDZK11P1 (Interaktionsprotein PDZK1), PI3 (Peptidase-Inhibitor), PSME2 (Aktivatoruntereinheit des Proteasom 2), SERPINB3 (Mitglied 3 des Serpin-Peptidase-Inhibitors), AKR1B10 (Mitglied B10 der Familie der Aldo-Keto-Reductasen), AKR1C1 (Mitglied C1 der Familie der Aldo-Keto-Reductasen), AKR1C2 (Mitglied C2 der Familie der Aldo-Keto-Reductasen), CYP1B1 (Cytochrom P450, Familie 1, Unterfamilie B, Polypeptid 1), FTH1P (schweres Polypeptid des Ferritin 1), FTL (leichtes Polypeptid des Ferritin 1), G6PD (Glucose-6-Phosphat-Dehydrogenase), GCLM (regulatorische Untereinheit der Glutamat-Cystein-Ligase), NQO2-(NAD(P)H-Dehydrogenase des Chinon s2), SLC7A11 (Mitglied 11 der Familie 7 der Träger der löslichen Moleküle), TXNRD1 (Thioredoxin-Reductase 1), UGT1A1 (Polypeptid A1 der Familie der UDP-Glucuronosyltransferase 1), UGT1A9 (Polypeptide A9 der Familie der UDP-Glucuronosyltransferase 1, YWHAZ (Zeta-Polypeptid des Aktivatorproteins von Tyrosin-3-Monooxygenase-/Tryptophan-5-Monooxygenase-Polypeptid), CD36 (CD36-Molekül), CYP1A1 (Cytochrom P450, Familie 1, Unterfamilie A, Polypeptid 1), GCLC (Katalysatoruntereinheit der Glutamat-Cystein-Ligase), HMOX1 (Hämoxygenase 1), NQO1 (NAD(P)H-Dehydrogenase der Chinone 1) und S100A8 (Kalzium bindendes Protein S100 A8), **gekennzeichnet dadurch, dass** die Hautprobe eine Probe von *in vitro* rekonstruierter Haut ist, und
c) Bestimmung der genannten Verbindung als Verbindung, die ein Sensibilisationspotential aufweist, wenn der Expressionsspiegel von mindestens sechs der genannten Gene über einem Schwellenwert liegt.

2. Bewertungsmethode gemäß Anspruch 1, in der der Expressionsspiegel jedes der genannten mindestens sechs Gene durch Messung des Expressionsspiegels des durch das genannte Gen oder ein Fragment dieses Gens kodierten Polypeptids oder durch Messung des Expressionsspiegels der m-RNA des genannten mindestens einen Gens oder eines Fragments dieses Gens bestimmt wird.

3. Bewertungsmethode gemäß Anspruch 1 oder 2, die einen zusätzlichen Schritt der Amplifikation der m-RNA oder cDNA jedes der genannten mindestens sechs Gene, deren ergänzende Sequenz oder ein Fragment von dieser umfasst.

4. Methode gemäß einem der Ansprüche 1 bis 3, in der der Schritt b) die Messung des Expressionsspiegels von mindestens 7, von mindestens 8, von mindestens 9, von mindestens 10, von mindestens 11, von mindestens 12, von mindestens 13, von mindestens 14, von mindestens 15, von mindestens 16, von mindestens 17, von mindestens 18, von mindestens 19, von mindestens 20, von mindestens 21, von mindestens 22, von mindestens 23, von mindestens 24 und vorzugsweise von mindestens 25 Genen umfasst, die aus der sich folgendermaßen zusammensetzenden Gruppe ausgewählt werden: BRAK (CXC-Chemokinligand 14), CTSS(Kathepsin S), DAPK2 (zelltodassoziierte Proteinkinase 2), FABP4 (Fettsäuren bindendes Protein 4), HSP27 (Hitzeschockprotein von 27kDa), IL18 (Interleukin 18), HSP90 (Hitzeschockprotein von 90kDa), IL1R2 (Rezeptor Typ II des Interleukin 1), TPSAB1 (Alpha-/Beta-Tryptase 1), CXCR1 (Alpha-Rezeptor des Interleukin 8), DEFB1 (Beta-Defensin 1), DHFR (Dihydrofolatreduktase), EHF (homologer Faktor ETS), IVL (Involucrin), KRT4 (Keratin 4), MELANA (Melan-A), NGAL (Gelatinase der Neutrophile), PDZK11P1 (Interaktionsprotein PDZK1), PI3 (Peptidase-Inhibitor), PSME2 (Aktivatoruntereinheit des Proteasom 2), SERPINB3 (Mitglied 3 des Serpin-Peptidase-Inhibitors), AKR1B10 (Mitglied B10 der Familie der Aldo-Keto-Reductasen), AKR1C1 (Mitglied C1 der Familie der Aldo-Keto-Reductasen), AKR1C2 (Mitglied C2 der Familie der Aldo-Keto-Reductasen), CYP1B1 (Cytochrom P450, Familie 1, Unterfamilie B, Polypeptid 1), FTH1P (schweres Polypeptid des Ferritin 1), FTL (leichtes Polypeptid des Ferritin 1), G6PD (Glucose-6-Phosphat-Dehydrogenase), GCLM (regulatorische Untereinheit der Glutamat-Cystein-Ligase), NQO2-(NAD(P)H-Dehydrogenase des Chinon s2), SLC7A11 (Mitglied 11 der Familie 7 der Träger der löslichen Moleküle), TXNRD1 (Thioredoxin-Reductase 1), UGT1A1 (Polypeptid A1 der Familie der UDP-Glucuronosyltransferase 1), UGT1A9 (Polypeptide A9 der Familie der UDP-Glucuronosyltransferase 1, YWHAZ (Zeta-Polypeptid des Aktivatorproteins von Tyrosin-3-Monooxygenase-/Tryptophan-5-Monooxygenase-Polypeptid), CD36 (CD36-Molekül), CYP1A1 (Cytochrom P450, Familie 1, Unterfamilie A, Polypeptid 1), GCLC (Katalysatoruntereinheit der Glutamat-Cystein-Ligase), HMOX1 (Hämoxygenase 1), NQO1 (NAD(P)H-Dehydrogenase der Chinone 1) und S100A8 (Kalzium bindendes Protein S100 A8) .

5. Methode gemäß einem der vorhergehenden Ansprüche, in der der Schritt b) zwischen 2 und 24 Stunden nach Schritt a), weiter bevorzugt zwischen 4 und 18 Stunden nach Schritt a), besonders bevorzugt zwischen 5 und 7 Stunden nach Schritt a) und bevorzugt nach 6 Stunden nach Schritt a) durchgeführt wird.

6. Methode zur Bewertung der Sensibilisationskraft einer Testverbindung, die die folgenden Schritte umfasst:
1) Gewinnung von mindestens einer Verdünnung der Testverbindung und
2) Messung des Sensibilisationspotentials der genannten mindestens einen Verdünnung der genannten Testverbindung durch eine Methode wie gemäß einem der vorhergehenden Ansprüche definiert.

7. Verwendung des Expressionsspiegels in einer Probe von *in vitro* rekonstruierter Haut von mindestens 6 Genen, die aus der sich folgendermaßen zusammensetzenden Gruppe ausgewählt werden: BRAK (CXC-Chemokinligand 14), CTSS (Kathepsin S), DAPK2 (zelltodassoziierte Proteinkinase 2), FABP4 (Fettsäuren bindendes Protein 4), HSP27 (Hitzeschockprotein von 27kDa), IL18 (Interleukin 18), HSP90 (Hitzeschockprotein von 90kDa), IL1R2 (Rezeptor Typ II des Interleukin 1), TPSAB1 (Alpha-/Beta-Tryptase 1), CXCR1 (Alpha-Rezeptor des Interleukin 8), DEFB1 (Beta-Defensin 1), DHFR (Dihydrofolatreduktase), EHF (homologer Faktor ETS), IVL (Involucrin), KRT4 (Keratin 4), MELANA (Melan-A), NGAL (Gelatinase der Neutrophile), PD2K11P1 (Interaktionsprotein PDZK1), PI3 (Peptidase-Inhibitor), PSME2 (Aktivatoruntereinheit des Proteasom 2), SERPINB3 (Mitglied 3 des Serpin-Peptidase-Inhibitors), AKR1B10 (Mitglied B10 der Familie der Aldo-Keto-Reductasen), AKR1C1 (Mitglied C1 der Familie der Aldo-Keto-Reductasen), AKR1C2 (Mitglied C2 der Familie der Aldo-Keto-Reductasen), CYP1B1 (Cytochrom P450, Familie 1, Unterfamilie B, Polypeptid 1), FTH1P (schweres Polypeptid des Ferritin 1), FTL (leichtes Polypeptid des Ferritin 1), G6PD (Glucose-6-Phosphat-Dehydrogenase), GCLM (regulatorische Untereinheit der Glutamat-Cystein-Ligase), NQO2-(NAD(P)H-Dehydrogenase des Chinon s2), SLC7A11 (Mitglied 11 der Familie 7 der Träger der löslichen Moleküle), TXNRD1 (Thioredoxin-Reductase 1), UGT1A1 (Polypeptid A1 der Familie der UDP-Glucuronosyltransferase 1), UGT1A9 (Polypeptide A9 der Familie der UDP-Glucuronosyltransferase 1, YWHAZ (Zeta-Polypeptid des Aktivatorproteins von Tyrosin-3-Monooxygenase-/Tryptophan-5-Monooxygenase-Polypeptid) zur Bewertung *in vitro* des sensibilisierenden Potentials einer Testverbindung.

## Claims

1. Method for evaluating the sensitising potential of a test compound, comprising steps for:
a) contacting a test compound with a skin sample
b) determining the level of expression of at least six genes chosen from the group consisting of: BRAK (CXC chemokine ligand 14), CTSS (Cathepsin S), DAPK2 (Death-associated protein kinase 2), FABP4 (Fatty acid binding protein 4), HSP27 (Heat shock 27kDa protein), IL18 (Interleukin 18), HSP90 (Heat shock 90kDa protein), IL1R2 (Interleukin 1 receptor type II), TPSAB1 (Tryptase alpha/beta 1), CXCR1 (Interleukin 8 receptor, alpha), DEFB1 (Defensin, beta 1), DHFR (Dihydrofolate reductase), EHF (ETS homologous factor), IVL (Involucrin), KRT4 (Keratin 4), MELANA (Melan-A), NGAL (Neutrophil gelatinase), PDZK1IP1 (PDZK1 interacting protein), PI3 (Peptidase inhibitor), PSME2 (Proteasome activator subunit 2), SERPINB3 (Serpin peptidase inhibitors, member 3), AKR1B10 (Aldo-keto reductase family, member B10), AKR1C1 (Aldo-keto reductase family, member C1), AKR1C2 (Aldo-keto reductase family, member C2), CYP1B1 (Cytochrome P450, family 1, subfamily B, polypeptide 1), FTH1P (Ferritin, heavy polypeptide 1), FTL (Ferritin, light polypeptide 1), G6PD (Glucose-6-phosphate dehydrogenase), GCLM (Glutamate-cysteine ligase modifier subunit), NQO2 (NAD(P)H dehydrogenase, quinone s2), SLC7A11 (Solute carrier family 7, member 11), TXNRD1 (Thioredoxin reductase 1), UGT1A1 (UDP glucuronosyltransferase family 1, polypeptide A1), UGT1A9 (UDP glucuronosyltransferase family 1, polypeptide A9), YWHAZ (Tyrosine 3-monooxygenase/tryptophan 5-monooxygenase polypeptide activation protein, zeta polypeptide), CD36 (CD36 molecule), CYP1A1 (Cytochrome P450, family 1, subfamily A, polypeptide 1), GCLC (Glutamate-cysteine ligase, catalytic subunit), HMOX1 (heme oxygenase 1), NQO1 (NAD(P)H dehydrogenase, quinone 1) and S100A8 (S100 calcium binding protein A8), **characterised in that** the skin sample is an in vitro reconstructed skin sample, and
c) determining said compound as having a sensitising potential if the level of expression of at least six of said genes is greater than a threshold value.

2. Evaluation method according to claim 1, wherein the level of expression of each of said at least six genes is determined by measuring the level of expression of the polypeptide coded by said gene or a fragment thereof, or by measuring the level of expression of the mRNA of said at least one gene or a fragment thereof.

3. Evaluation method according to any of claims 1 or 2, comprising an additional step for amplifying the mRNA or cDNA of each of said at least six genes, the complementary sequence thereof or a fragment thereof.

4. Method according to any of claims 1 to 3, wherein step b) comprises the measurement of the expression of at least 7, at least 8, at least 9, at least 10, at least 11, at least 12, at least 13, at least 14, at least 15, at least 16, at least 17, at least 18, at least 19, at least 20, at least 21, at least 22, at least 23, at least 24 and more preferentially at least 25 genes chosen from the group consisting of: BRAK (CXC chemokine ligand 14), CTSS (Cathepsin S), DAPK2 (Death-associated protein kinase 2), FABP4 (Fatty acid binding protein 4), HSP27 (Heat shock 27kDa protein), IL18 (Interleukin 18), HSP90 (Heat shock 90kDa protein), IL1R2 (Interleukin 1 receptor type II), TPSAB1 (Tryptase alpha/beta 1), CXCR1 (Interleukin 8 receptor, alpha), DEFB1 (Defensin, beta 1), DHFR (Dihydrofolate reductase), EHF (ETS homologous factor), IVL (Involucrin), KRT4 (Keratin 4), MELANA (Melan-A), NGAL (Neutrophil gelatinase), PDZK1IP1 (PDZK1 interacting protein), PI3 (Peptidase inhibitor), PSME2 (Proteasome activator subunit 2), SERPINB3 (Serpin peptidase inhibitors, member 3), AKR1B10 (Aldo-keto reductase family, member B10), AKR1C1 (Aldo-keto reductase family, member C1), AKR1C2 (Aldo-keto reductase family, member C2), CYP1B1 (Cytochrome P450, family 1, subfamily B, polypeptide 1), FTH1P (Ferritin, heavy polypeptide 1), FTL (Ferritin, light polypeptide 1), G6PD (Glucose-6-phosphate dehydrogenase), GCLM (Glutamate-cysteine ligase modifier subunit), NQO2 (NAD(P)H dehydrogenase, quinone s2), SLC7A11 (Solute carrier family 7, member 11), TXNRD1 (Thioredoxin reductase 1), UGT1A1 (UDP glucuronosyltransferase family 1, polypeptide A1), UGT1A9 (UDP glucuronosyltransferase family 1, polypeptide A9), YWHAZ (Tyrosine 3-monooxygenase/tryptophan 5-monooxygenase polypeptide activation protein, zeta polypeptide), CD36 (CD36 molecule), CYP1A1 (Cytochrome P450, family 1, subfamily A, polypeptide 1), GCLC (Glutamate-cysteine ligase, catalytic subunit), HMOX1 (heme oxygenase 1), NQO1 (NAD(P)H dehydrogenase, quinone 1) and S100A8 (S100 calcium binding protein A8).

5. Method according to any of the above claims, wherein step b) is conducted between 2 and 24 hours after step a), more preferably between 4 and 18 hours after step a), particularly preferably between 5 and 7 hours after step a) and most preferably 6 hours after step a).

6. Method for evaluating the sensitising strength of a test compound comprising the following steps:
1) obtaining at least one dilution of the test compound, and
2) measuring the sensitising potential of said test compound at said at least one dilution by means of a method as defined according to any of the above claims.

7. Use of the level of expression, in an in vitro reconstructed skin sample, of at least 6 genes chosen from the group consisting of: BRAK (CXC chemokine ligand 14), CTSS (Cathepsin S), DAPK2 (Death-associated protein kinase 2), FABP4 (Fatty acid binding protein 4), HSP27 (Heat shock 27kDa protein), IL18 (Interleukin 18), HSP90 (Heat shock 90kDa protein), IL1R2 (Interleukin 1 receptor type II), TPSAB1 (Tryptase alpha/beta 1), CXCR1 (Interleukin 8 receptor, alpha), DEFB1 (Defensin, beta 1), DHFR (Dihydrofolate reductase), EHF (ETS homologous factor), IVL (Involucrin), KRT4 (Keratin 4), MELANA (Melan-A), NGAL (Neutrophil gelatinase), PDZK1IP1 (PDZK1 interacting protein), PI3 (Peptidase inhibitor), PSME2 (Proteasome activator subunit 2), SERPINB3 (Serpin peptidase inhibitors, member 3), AKR1B10 (Aldo-keto reductase family, member B10), AKR1C1 (Aldo-keto reductase family, member C1), AKR1C2 (Aldo-keto reductase family, member C2), CYP1B1 (Cytochrome P450, family 1, subfamily B, polypeptide 1), FTH1P (Ferritin, heavy polypeptide 1), FTL (Ferritin, light polypeptide 1), G6PD (Glucose-6-phosphate dehydrogenase), GCLM (Glutamate-cysteine ligase modifier subunit), NQO2 (NAD(P)H dehydrogenase, quinone s2), SLC7A11 (Solute carrier family 7, member 11), TXNRD1 (Thioredoxin reductase 1), UGT1A1 (UDP glucuronosyltransferase family 1, polypeptide A1), UGT1A9 (UDP glucuronosyltransferase family 1, polypeptide A9) and YWHAZ (Tyrosine 3-monooxygenase/tryptophan 5-monooxygenase polypeptide activation protein, zeta polypeptide) for the in vitro evaluation of the sensitising potential of a test compound.
